# EUROPEAN PATENT APPLICATION

(11) **EP 1 038 958 A1**
(43) Date of publication of application: **27.09.2000**
(21) Application number: 98959200.1
(22) Date of filing: 14.12.1998
(51) Int. Cl.: C12N 15/12, C12N 15/63, C12N 5/10, C12P 21/02, C12P 21/08, C07K 14/47, C07K 16/18, A61K 38/17, A61K 39/395, G01N 33/53

(54) **HUMAN Nap1 PROTEIN**

(30) Priority: 15.12.1997 JP 36318397
(71) Applicant: KYOWA HAKKO KOGYO CO., LTD., Chiyoda-ku, Tokyo 100-8185 (JP); Sakaki, Yoshiyuki, Yokohama-shi, Kanagawa 236-0045 (JP)
(72) Inventor: SAKAKI, Yoshiyuki, Yokohama-shi, Kanagawa 236-0045 (JP)
(74) Representative: VOSSIUS & PARTNER
(86) International application number: JP9805646
(87) International publication number: WO9931239

(57) **Abstract**

A human Nap1 protein having the amino acid sequence represented by SEQ ID NO. 2 or a protein having an amino acid sequence which includes substitution, deletion or addition of one or more amino acids of the amino acid sequence represented by SEQ ID NO. 2 and having apoptosis-suppressing activities.

## Description

### Technical Field

This invention relates to a human Nap1 protein, a DNA encoding said protein, a process for producing said protein, an antibody recognizing said protein and the use of said protein.

### Background Art

Alzheimer's disease is a progressive neurodegenerative disorder of the brain, which is one of the causes of senile dementia. The Alzheimer's disease is divided into familial one considered to be caused by genetic mutation and sporadic one, and the latter accounts for the majority. Its pathological features in both the familial case and the sporadic case are cerebral atrophy caused by neuronal loss and massive neuronal cell death, neuronal degeneration called neurofibrillary tangles, amyloid fibril deposition in the intercellular spaces called senile plaque, etc. Although many studies have been made of factors participating in the onset of said disease, the biochemical mechanism leading to these disorders has not yet been elucidated well.

Familial Alzheimer's disease is considered to be caused by genetic mutation. And, from the genetic analysis of the familial Alzheimer's disease, ß -amyloid precursor protein [*Nature*, 325, 733 (1987)], presenilin 1 [*Nature* 375, 754 (1995) and presenilin 2 [*Science,* 269, 973 (1995)] have been reported as Alzheimer's disease-related genes. Besides, as a gene considered to be a risk factor of the onset, the type 4 allele out of allele polymorphisms of apolipoprotein E (apo E) [*Science* 261, 921 (1993)] have been reported.

Although experiments using normal cells have brought a report that apoptosis was induced when a mutated ß -amyloid precursor protein gene was introduced and expressed in a cell [*Science,* 272, 1349 (1996)], a report that cell death was induced when ß -amyloid peptide derived from ß -amyloid precursor protein was administered to neuronal cells [*Science*, 245, 417 (1989)], a report that apoptosis was induced by a peptide corresponding to a part of ß -amyloid peptide [*Proceedings of the National Academy of Sciences of the United States of America*, 90, 7951 (1993)], there are still unclear points about how the above gene participates in neuronal cell death and the onset of Alzheimer's disease.

Over against this, it has been known that apoptosis occurs in neuronal cells of a patient with Alzheimer's disease [*Experimental Neurology*, 133, 225 (1995)]. If such apoptosis in neuronal cells is brought by the same mechanism as that in which ß -amyloid precursor protein or the like participates, it is considered that the neuronal cell death is repressed by repressing apoptosis to make the prevention and the treatment of Alzheimer's disease possible.

On the other hand, sporadic Alzheimer's disease accounts for the majority of Alzheimer's disease, the onset mechanism of which has scarcely been known. It is not also unclear whether the above mechanism of neuronal cell death with which the above gene associates also participates in the sporadic Alzheimer's disease or not.

By the way, an Nck associated protein (hereinafter abbreviated to Nap) is a protein which was identified as a 125-kDa protein in a bovine brain extract and which specifically but indirectly associates with the 1st SH3 domain of an intracellular signal transduction molecule Nck [*Biochemical and Biophysical Research Communication,* 219, 509 (1996)].

Before now, a mouse Nap1 and a cDNA encoding a rat Nap1 has been isolated. That is, based on an inference from the partial amino acid sequence of a Nap1 protein purified from a bovine brain, the mouse cerebellum cDNA clone H19 [*European Journal of Neuroscience*, 2, 704 (1990)] is considered to be a mouse Nap1 partial cDNA clone, then a rat brain cDNA library was screened using the cDNA of said H19 as a probe. As a result of it, a full length rat Nap1 cDNA was obtained. This cDNA encoded rat Nap1 protein comprising 1128 amino acids [*Biochem. Biphys. Res. Commun*., 219, 509 (1996)].

Incidentally, as proteins having homology to rat Nap1, gene expression products of human Hem-1 cDNA cloned as a gene which is specifically expressing in a human hemocyte [*Biochimica et Biophysica Acta*, 1090, 241 (1991)], a Drosophila gene dhem-2 which is considered to play an important role in oogenesis and early embryogenesis [*Journal of Molecular Biology*, 251, 41 (1995)] and a rat Hem-2 cDNA obtained from a rat brain cDNA library using H19 cDNA as a probe [*J. Mol. Biol*., 251, 41 (1995)] are known. The amino acid sequence homologies of respective proteins with the rat Nap1 are such high values as 59% in the human Hem-1; 60% in the Drosophila dhem-2 and 94% in the rat Hem-2.

The Nck with which Nap1 is associated is an intracellular protein comprising 377 amino acids and has an SH2 domain and three SH3 domains [*Nucleic Acids Research,* 18, 1048 (1990)]. Generally, it is considered that the SH2 domain has a property of binding to a domain containing phosphorylated tyrosine and the SH3 domain has a property of binding to a proline-rich domain, and molecules having these domains are considered to participate in intracellular signal transduction. That is, a large number of growth factor receptors intracellularly carry a domain having tyrosine kinase activities, and it is considered that the tyrosine kinase is activated by binding to a ligand to phosphorylate tyrosine residues of own or other molecules to thereby start the signal transduction. For example, it is reported that Nck, via its SH2 domain, binds to an autophosphorylated tyrosine of a tyrosine kinase type receptor of a cell growth factor such as epidermal growth factor (EGF), platelet derived growth factor (PDGF), vascular endothelial growth factor (VEGF), hepatocyte growth factor (HGF) or the like to undergo phosphorylation [*Proc. Natl. Acad. Sci. USA*, 89, 8894 (1992); *Molecular & Cellular Biology*, 12, 5824 (1992); *Journal of Biological chemistry*, 270, 6729 (1995); *Biochemistry & Molecular Biology International*, 36, 465 (1995)].

As molecules binding to the Nck SH3 domain, PAK (p21ras-activated kinase), p21ras activation factor Sos by guanine nucleotide exchange, NAK (Nck-associated kinase) as a serine/threonine kinase, c-cb1 as a proto-oncogene to be phosphorylated upon simulation of various receptors and SAKAP II (Src A box Nck-associated protein II) as a causative gene of Whiskoff-Aldrich syndrome have been reported [*J. Biol. Chem*., 270, 22731 (1995); *Mol. Cell. Biol*., 15, 1169 (1995); J. Biol Chem., 270, 7359 (1995), J. Biol. Chem., 269, 17363 (1994); and *Mol. Cell. Biol*., 15, 5725 (1995)].

Judging from such associated molecules as above, it is considered that Nck is playing an important role mainly in mitogenic signal transduction pathways of growth factors, oncogenes, etc. Actually, it is reported that, when the Nck was overexpressed in NIH3T3 cells or 3Y1 cells as fibroblast cells, said cells are oncogenically transformed [*Mol. Cell. Biol*., 12, 5824 (1992) and *Mol. Cell. Biol*., 12, 5834 (1992)]. Besides, PC12 cells are differentiated into a neuronal cells, arrested the growth and made to project neurites by a nerve-derived growth factor (NGF). It is reported that, as a result of overexpressing the Nck in PC12 cells, the differentiation was blocked and the proliferation was continued even by administering the NGF to the cells [*Oncogene*, 12, 2351 (1996)]. Furthermore, there also is a report that Nck activated the promoter of transcription factor fos as a proto-oncogene [*Mol. Cell. Biol*., 15, 1169 (1995)].

Therefore, it is considered that Nap1 which is a molecule specifically associated with the Nck SH3 domain also plays a role in the cell proliferation system.

However, its direct function and action are unclear. In addition, there is neither a report showing the relation between Nap1 and Alzheimer's disease nor a report that Nap1 has an inhibitory effect on apoptosis. Furthermore, with respect to the information about human Nap1 gene and protein, only some EST (Expressed Sequence Tag; collection of information about 3'-end and 5'-end nucleotide sequences of a large number of cDNA clones in a cDNA library with redundancy), that is nucleotide sequences of the 5'-end moieties of human cDNA clones, which show homology to rat Hem-2 or mouse H19, are reported in nucleotide sequence data base Genbank, its whole structure is still unknown.

Therefore, the elucidation of genes and the like related to Alzheimer's disease is anticipated for the diagnosis, the prevention and the treatment of Alzheimer's disease.

### DISCLOSURE OF THE INVENTION

The present invention purposes to provide a human Nap1 protein, a DNA encoding said protein, a process for producing said protein, an antibody recognizing said protein and the use of said protein.

The present inventors, as a result of making intensive studies for solving the above problems, succeeded in isolating human Nap1 (Nck associated protein 1) related to Alzheimer's disease and cloning a DNA encoding said protein, thereby completing the present invention.

That is, the present invention relates to the following (1) to (19).
(1) A human Nap1 protein having the amino acid sequence represented by SEQ ID NO. 2 or a protein having an amino acid sequence which includes substitution, deletion or addition of one or more (preferably one or several) amino acids of the amino acid sequence represented by SEQ ID NO. 2 and having apoptosis-suppressing activities.
   The above deletion, substitution or addition of amino acids can be carried out according to the site-specific mutagenesis which had been publicly known before the present application, and the term 'one or several amino acids' means amino acids in such number as can be deleted, substituted or added by the site-specific mutagenesis.
   Such a protein comprising an amino acid sequence containing one or several deleted, substituted or added amino acids and having apoptosis suppression activities can be prepared according to the methods described in Molecular Cloning, A Laboratory Manual, Second Edition, Cold Spring Harbor Laboratory Press (1989) (hereinafter abbreviated to Molecular Cloning Second Edition); Current Protocols in Molecular Biology, Supplement 1 to 38, John Wiley & Sons (1987 - 1997) (hereinafter abbreviated to Current Protocols in Molecular Biology); *Nucleic Acid Research*, 10, 6487 (1982); *Proc. Natl. Acad. Sci. USA,* 79, 6409 (1982); *Gene*, 34, 315 (1985); *Nucleic Acids Research*, 13, 4431 (1985); *Proc. Natl. Acad. Sci USA,* 82, 488 (1985); *Proc. Natl. Acad. Sci. USA,* 81, 5662 (1984); *Science,* 224, 1431 (1984); PCT WO85/00817 (1985); *Nature*, 316, 601 (1985); etc. Besides, such a protein as above can be prepared also by using commercially available kits (Site-Directed Mutagenesis Kit of CLONETECH and the like) other than the above methods.
(2) A DNA encoding the protein described in (1) above.
(3) A DNA having the nucleotide sequence represented by SEQ ID NO. 1. (4) A DNA capable of hybridizing with the nucleotide sequence of the DNA described in (2) or (3) above under stringent conditions and encoding a protein having apoptosis-suppressing activities.
   'A DNA capable of hybridizing under stringent conditions and having apoptosis-suppressing activities' described above means a DNA which is obtained by using the DNA described in (2) or (3) above as a probe and employing colony hybridization method, plaque hybridization techniques, Southern blot hybridization techniques or the like. Specifically, a DNA obtained by carrying out hybridization at 65°C in the presence of 0.7M to 1.0M NaCl using a filter on which a colony- or plaque-derived DNA is immobilized and subsequent identification by washing the filter at 65°C with a SSC (saline sodium citrate) solution with 0.1 to 2-fold concentration (the composition of the SSC solution with 1-fold concentration comprises 150mM sodium chloride and 15mM sodium citrate) can be given.
   The hybridization can be carried out in compliance with the methods described in experimented manuals such as Molecular Cloning, Second Edition; Protocol in Molecular Biology; DNA Cloning 1: Core Techniques, A Practical Approach, Second Edition, Oxford University Press (1995); etc.
   As specific examples of the hybridizable DNA, DNAs having at least 60% or more, preferably 80% or more, more preferably 95% or more homology with the nucleotide sequence represented by SEQ ID NO. 1 can be enumerated.
(5) A recombinant vector comprising the DNA described in any one of (2) to (4) above and a vector
(6) A transformant to be obtained by introducing the vector described in (5) above into a host cell.
(7) A process for producing the foregoing protein, characterized by culturing the transformant described in (6) above in a medium to produce and accumulate the protein described in (1) above in the culture and harvesting the protein from the culture to be obtained.
(8) A therapeutic agent for Alzheimer's disease, which contains the protein described in (1) above as an active ingredient.
(9) An oligonucleotide having a nucleotide sequence comprising continuous 5 to 60 residues out of the nucleotide sequence of the DNA described in any one of (2) to (4) above, or an oligonucleotide having a sequence complementary to said oligonucleotide.
(10) An oligonucleotide described in (9) above, which has a nucleotide sequence represented by SEQ ID NO. 17 or 18.
(11) An oligonucleotide described in (9) above, which has a nucleotide sequence represented by SEQ ID NO. 26 or 28.
(12) A method for detecting an mRNA of a human Nap1 gene, which uses the oligonucleotide described in (9) or (10) above.
(13) A diagnostic reagent for Alzheimer's disease, which contains the oligonucleotide described in (9) or (10).
(14) A method for repressing the transcription of the human Nap1 gene or the translation of the mRNA, which uses an oligonucleotide described in (9) or (11) above.
(15) A therapeutic composition for apoptosis-participating diseases, which contains the oligonucleotide described in (9) or (11) above.
(16) An antibody recognizing the protein described in (1) above.
(17) A method for immunologically detecting the protein described in (1) above, which uses the antibody described in (16) above.
(18) A diagnostic reagent for Alzheimer's disease, which contains the antibody described in (16) above.
(19) A therapeutic composition for apoptosis-participating diseases, which contains the antibody described in (16) above as an active ingredient.

As examples of the protein of the present invention, human Nap1 protein having the amino acid sequence represented by SEQ ID NO. 2 or a protein having an amino acid sequence which includes substitution, deletion or addition of one or more amino acids of the amino acid sequence represented by SEQ ID NO. 2 and having apoptosis-suppressing activities can be given.

In addition, the DNA of the present invention includes a DNA having a part of nucleotide sequence of the said DNA described above or a DNA having a sequence complementary to said DNA. As example of the DNA having a part of nucleotide sequence of the present DNA, an oligonucleotide having the identical nucleotide sequence as continuous 5 to 60 residues, preferably 10 to 40 residues of the nucleotide sequence represented by SEQ ID NO. 1 can be given, and, as an example of the DNA having a sequence complementary to the DNA having a part of nucleotide sequence of the present DNA, an antisense oligonucleotide of said oligonucleotide can be given. A DNA having a nucleotide sequence represented by SEQ ID NO. 17, 21, 23 or 24 can be given as an example of said oligonucleotide, and a DNA having a nucleotide sequence represented by SEQ ID NO. 18, 22, 25, 26 or 28 can be given as an example of said antisense oligonucleotide.

Hereinafter the present invention will be described in detail. Incidentally, ordinary methods described in this text means those described in experimental manuals such as 'Molecular Cloning, Second Edition' and the like.

The specification of the present invention includes the contents described in the specification and/or the drawings of Japanese Patent Application No. 363,183/1997 as the basis of the priority of the present application.

### 1. Preparation of DNA Relevant to Alzheimer's Disease

In order to elucidate the molecular mechanism of sporadic Alzheimer's disease, it is important to find out molecules participating in its onset. Exploration of a gene showing a different expression pattern between the brain of a sporadic Alzheimer's disease patient and that of a non-Alzheimer's disease person, that is, a gene showing quantitative change affords a clue to the discovery of molecules participating in the onset thereof. Then, taking notice of the change in expression of mRNA in the brain of a sporadic Alzheimer's disease patient and that of a non-Alzheimer's disease person, a DNA related to the onset of Alzheimer's disease is prepared employing the differential display technique [*Science* 257, 967 (1992); *FEBS Letters*, 351, 231 (1994)].

That is, the total RNAs are prepared from the brain of a sporadic Alzheimer's disease patient and that of a non-Alzheimer's disease person first of all. As examples of the method for preparing the total RNA from brain tissues, the guanidine thiocyanate-cesium trifluoroacetate method [*Methods in Enzymology*, 154, 3 (1987)], the AGPC method [*Experimental Medicine*, 2 1937 (1991)], etc. can be given.

In addition, as examples of the method for preparing mRNA as a poly (A)+RNA from the total RNA, the oligo(dT) immobilized cellulose column technique (Molecular Cloning, Second Edition), etc. can be given.

Furthermore, it is also possible to prepare mRNA directly from brain tissues by using a kit such as Fast Track mRNA Isolation Kit (manufactured by Invitrogen), Quick Prep mRNA Purification Kit (manufactured by Pharmacia) or the like.

From each of the above RNAs extracted from brain tissues, cDNAs are synthesized using anchor primers according to ordinary methods, and subsequently each of these cDNAs are subjected to PCR using the anchor primers and arbitrary primers to thereby amplify the cDNAs.

The anchor primer means a primer prepared by adding an oligonucleotide exclusive of thymidine such as adenine, guanine or cytosine to the 3'-end of an oligo(dT) sequence which hibridizes with the 3'-end polyA sequence of a mRNA, as examples of which oligonucleotides having the nucleotide sequences represented by SEQ ID NOS. 4 to 6 can be given.

The arbitrary primer means an oligonucleotide which can amplify against varieties of cDNA sequences, and can obtain a large number of cDNA amplification fragment by only one reaction, as examples of which OPA-1 to -20, OPB-1 to -20, OPC-1 to -20, OPD-1 to -20, etc. manufactured by Operon Technologies can be given. The length of an arbitrary primer is preferably on the order of 10 to 20 bases.

Subsequently, each of the above cDNAs amplified by PCR is electrophoresed on a polyacrylamide gel, and the quantity of amplification of each band can be determined by fluorescently staining each amplified cDNA with a DNA-specific fluorescent stain such as SYBR Green I or the like and measuring the intensity of fluorescence thereof using a fluorimager. Besides, when an anchor primer previously labeled fluorescently at the 5' end is used in PCR, the intensity of fluorescence of a band can be determined just after the electrophoresis without using a fluorescent stain. The fluorescent label of the 5'-end of the primer can be carried out using fluorescein isothiocyanate according to an ordinary method.

The intensity of fluorescence of respective bands are compared, the gel corresponding the position of a band with a changed fluorescence intensity is cut out, the gel cut out is placed in a reaction solution to carry out PCR, and whereby a DNA fragment in the gel is amplified.

Said amplified DNA fragment is ligated into a vector according to an ordinary method as it is or after blunting its end with pfuDNA polymerase and then analyzed employing ordinary used nucleotide sequence analysis methods, for example, the dideoxy method of Sanger a al. [*Proc. Natl. Acad. Sci. USA*, 74, 5463 (1977)] or a nucleotide sequence analyzer such as a DNA Sequencer 373A (manufactured by Perkin Elmer) or the like, and whereby the nucleotide sequence of said DNA is determined.

As examples of the vector in which said amplified DNA fragment is ligated, pCR-Script Amp [manufactured by Stratagene; *Stratagies*, 5, 6265 (1992)], pT7Blue T-Vector (manufactured by Novagen), pCR2.1 [manufactured by Invitrogen; *Bio/Technology*, 9, 657 (1991)], pCR-TRAP (manufactured by GenHunter), pTA (manufactured by Nippon Gene K.K.), pNoTA T7 (manufactured by 5'→3', Inc.), etc. can be given.

The novelty of thus determined nucleotide sequence can be confirmed by searching nucleotide sequence data bases such as GenBank, EMBL, DDBJ, etc. using a homology-searching program such as BLAST or the like to find out the absence of a nucleotide sequence showing obvious homology with a nucleotide sequence in the data bases, as considered identical.

As examples of the DNA obtained as above, a DNA having the sequence represented by SEQ ID NO. 3 and the like can be given.

In case that the DNA obtained according to the above method is a partial DNA fragment of the cDNA corresponding to the mRNA of the gene the expression of which is changed in the brain of a Alzheimer's disease patient with, a full length cDNA can be obtained according to the methods of (1) to (3) set forth below.
(1) Utilization of cDNA library
   By carrying out screening by means of hybridization with various cDNA libraries using the above DNA fragment as a probe, a full length cDNA can be obtained.
   Hereinafter, a method for making a cDNA library will be described.
   As examples of the method for making a cDNA library, methods described in Molecular Cloning, Second Edition; Current Protocols in Molecular Biology, Supplement 1 to 34, Greene Publishing Associates and Wiley-Interscience, 1987-1996 Edition (hereinafter abbreviated to Current Protocols in Molecular Biology, Supplement 1 to 34); etc. or methods employing commercially available kits, for example SuperScript Plasmid System for cDNA Synthesis and Plasmid Cloning (manufactured by Life Technologies) or ZAP-cDNA Synthesis Kit (manufactured by Stratagene) can be given. In addition, a commercially available article of the very cDNA library, a human brain cDNA library supplied by CLONETECH, etc. are also utilizable.
   As a cloning vector for making a cDNA library, any of a phage vector, a plasmid vector, etc. can be used as far as it is able to replicate autonomously in the *Escherichia coli* strain K12. Specifically, ZAP Express [manufactured by Stratagene; *Strategies*, 5, 58 (1992)], pBluescript II SK(+) [*Nucleic Acids Research,* 17, 9494 (1989)], Lambda ZAP II (manufactured by Stratagene), λgt10, λgt11 [DNA *Cloning*, A Practical Approach, 1, 49 (1985)], λTriplEx (manufactured By CLONETECH), λExCell (manufactured by Pharmacia), pT7T318U (manufactured by Pharmacia, Inc.), pcD2 [*Mol. Cell. Biol*., 3, 280 (1983)], pUC18 [*Gene*, 33, 103 (1985)], etc. can be enumerated.
   As a host microorganism, any microorganism can be used as far as it is a microorganism belonging to *Escherichia coli*. Specifically, *Escherichia coli* XL1-Blue MRF' [manufactured by Stratagene; *Strategies*, 5, 81(1992)], *Escherichia coli* C600 [*Genetics*, 39, 440 (1954)], *Escherichia coli* Y1088 [*Science* 222, 778 (1983)], *Escherichia coli* Y1090 [*Science* 222, 778 (1983)], *Escherichia coli* NM522 [*J. Mol. Biol*., 166, 1 (1983)], *Escherichia coli* K802 [*J. Mol. Biol.*, 16, 118 (1966)], *Escherichia coli* JM105 [*Gene* 38, 275 (1985)], etc. are used.
   The selection of a cDNA clone from the cDNA library can be carried out according to colony hybridization method or plaque hybridization method using an isotope- or a fluorescence-labeled probe (Molecular Cloning, Second Edition).
   The aimed DNA can be obtained from the selected clone according to a generally known method.
(2) A cDNA is synthesized from a mRNA according to the above method, adapters are added to the both end of said cDNA, and a 5'-end cDNA and a 3'-end cDNA can be obtained respectively from the amplification fragment by 5'-RACE (rapid amplification of cDNA ends) and 3'-RACE [Proc. Natl. Acad. Sci. USA, 85, 8998 (1988)] according to which PCR is carried out using primers based on the nucleotide sequences of said adapters and that of the amplification fragment. And, the aimed DNA can be obtained by ligating the obtained cDNA to the amplification fragment.
(3) The aimed DNA can be obtained by PCR (PCR Protocols, Academic Press, 1990) using primers prepared based on the nucleotide sequences of the 5'-end and 3'-end of the full length cDNA obtained according to the method of (1) or (2) and a cDNA synthesized from a mRNA or a cDNA library as a template.

The nucleotide sequences of the DNAs obtained according to the above methods can be determined by the above nucleotide sequencing method. Also the novelty of said sequences can be confirmed according to the above method.

As examples of the DNA having a novel nucleotide sequence confirmed according to the above method, a DNA having the sequence represented by SEQ ID NO. 1 and the like can be given. In addition, the amino acid sequence of the human Nap1 protein according to the present invention is given as an example in SEQ ID NO. 2, in which variations such as deletion, substitution, addition and the like of one or more (preferably one or several) amino acids may occur in said amino acid sequence as far as a protein comprising this amino acid sequence has apoptosis-suppressing activities. For example, a protein having a sequence which has the deletion of the 1st methionine of the amino acid sequence represented by SEQ ID NO. 2 is also included in the protein according to the present invention as far as it has apoptosis-suppressing activities.

It is also possible to prepare the aimed DNA by carrying out chemical synthesis using a DNA synthesizer on the basis of the determined DNA nucleotide sequence. As examples of the DNA synthesizer, a thiophosphite method-applying DNA synthesizer (manufactured by Shimadzu Corporation), a phosphor amidite method-applied DNA synthesizer model 392 (manufactured by Perkin-Eliner), etc. can be given.

An oligonucleotide having a partial sequence of a DNA participating in apoptosis and an antisense oligonucleotide can be obtained by using said DNA and DNA fragment according to an generally known method or using a DNA synthesizer.

As examples of said oligonucleotide or antisense oligonucleotide, a sense primer corresponding to the 5'-end nucleotide sequence, an antisense primer corresponding to the 3'-end nucleotide sequence of the part of nucleotide sequence of a mRNA for detection. However, a base corresponding to uracyl in the mRNA is thymidine in the oligonucleotide primer.

As the sense primer and the antisense primer, oligonucleotides with 5 to 60 residues oligonucleotides whose respective melting temperatures (Tm) and number of bases are not different extremely are given, and those with 10 to 40 residues are preferable.

As examples of the sense oligonucleotide or the antisense oligonucleotide having a part of sequence of the human Nap1 DNA, as to the sense oligonucleotide the one represented by SEQ ID NO. 17, 21, 23 or 24 and as to the antisense oligonucleotide the one represented by SEQ ID NO. 18, 22, 25, 26 or 28 can be given.

In addition, also a derivative of said nucleotide can be used in the present invention, as examples of which a methyl-modified one and a phosphorothioate-modified one of said nucleotide can be enumerated.

### 2. Preparation of Human Nap1 Protein

In order to express the human Nap1 DNA obtained according to the method described in 1 above in a host cell, methods described in Molecular Cloning, Second Edition; Current Protocols in Molecular Biology, Supplement 1 to 34; etc. can be adopted. That is, a transformant expressing the protein of the present invention can be obtained by inserting a DNA encoding the protein of the present invention downstream of a promoter in an appropriate expression vector to construct a recombinant vector and then introducing the same into a host cell.

As the host cell, bacteria, yeast, animal cells, insect cells, etc. can be used as far as they can express the aimed gene.

As the expression vector, a vector which is able to replicate autonomously or is capable of being integrated into the chromosome in the above host cell and which contains a promoter at a site where the protein of the present invention can be transcribed is used.

In case of using a prokaryotic cell such as a bacterium or the like as a host cell, it is preferred that the human Nap1 expression vector should be able to replicate autonomously in the prokaryotic cell and simultaneously be composed of a promoter, a ribosome binding sequence, a human Nap1 gene and a transcription termination sequence. In addition, a gene controlling the promoter may be contained.

As examples of the expression vector, pKK233-2 (manufactured by Pharmacia), pSE280 (manufactured by Invitrogen), pGEMEX-1 (manufactured by Promega), pQE-8 (manufactured by QIAGEN), pKYP10 (Japanese Patent Application Laid open No. 110,600/1983), pKYP200 [*Agricultural Biological Chemistry*, 48, 669 (1984)], pLSA1 [*Agric. Biol. Chem*., 53, 277 (1989)], pGEL1 [*Proc. Natl. Acad. Sci. USA*, 82, 4306 (1985)]. pBluescript II SK(-) (manufactured by Stratagene), pTrs30 [prepared from *Escherichia coli* JM109/pTrS30 (FERM BP-5407), pTrs 32 [prepared from *Escherichia coli* JM 109/pTrs 32 (FERM BP-5408)], pGKA2 [prepared from *Escherichia coli* IGKA (FERM BP-6798), see Japanese Patent Application Laid open No. 221,091/1985], pTerm2 (see Japanese Patent Application Laid open No. 22,979/1991, US4686191, US4939094, US5160735), pGEX (manufactured by Pharmacia), pET System (manufactured by Novagen), pSupex, etc. can be given.

As the promoter, any promoter may be employed as far as it can be expressed in a host cell such as *Escherichia coli* or the like, as examples of which promoters originated from *Escherichia coli*, phages, etc. such as *trp* promoter (P*trp*), *lac* promoter, P_{L} promoter, P_{R} promoter, T7 promoter, etc. can be given. In addition, artificially designed or modified promoters such as a promoter composed of two P*trp* connected in series (P*trp* × 2), *tac* promoter, *lac*T7 promoter, *let*I promoter, etc. can be used also.

As the ribosome binding sequence, a plasmid having a suitably regulated spacing (e.g., 6 to 18 bases) between the Shine-Dalgano sequence and the starting codon is preferably used.

In the present recombinant vector, it is preferable that a transcription termination sequence is positioned just downstream from the structural gene though said sequence is not always necessary for the expression of the present DNA.

As a host cell, microorganisms belonging to the genera *Escherichia, Serratia, Corynebacterium, Brevibacterium, Pseudomonas, Bacillus*, etc., for example, *Escherichia coli* XL1-Blue, *Escherichia coli* XL2-Blue, *Escherichia coli* DH1, *Escherichia coli* MC 1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* NY49, *Bacillus subtilis, Bacillus amyloliquefacines, Brevibacterium immariophilum* ATCC14068, *Brevibacterium saccharolyticum* ATCC14066, *Brevibacterium flavum* ATCC14067, *Brevibacterium lactofermentum* ATCC13869, *Corynebacterium glutamicum* ATCC13032, *Corynebacterium acetoacidophilum* ATCC13870, *Microbacterium ammoniaphilum* ATCC15354, etc. can be given.

As an introduction method of the recombinant vector, any method can be adopted as far as it is a method for introducing a DNA into the above host cell, as examples of which a method using calcium ions [*Proc. Natl. Acad. Sci. USA*, 69, 2110 (1972)], the protoplast method (Japanese Patent Application Laid open No. 248,394/1988), etc. can be given.

In case of using a yeast strain as a host cell, YEp13 (ATCC37115), Yep24 (ATCC37051), Ycp50 (ATCC37419), etc. can be used as examples of an expression vector.

As a promoter, any promoter may be used as far as it can be expressed in the yeast strain, as examples of which a promoter of a glycolytic gene such as hexokinase or the like, gal 1 promoter, gal 10 promoter, heat shock protein promoter, MF α 1 promoter, CUP 1 promoter, etc. can be given.

As a host cell, *Saccharomyces cerevisiae, Schizosaccharomyces pombe, Kluyveromyces lactis, Trichosporon pullulans, Schwanniomycess alluvius,* etc. can be enumerated.

As an introduction method of the recombinant vector, any method can be adopted as far as it is a method for introducing a DNA into yeast, as examples of which electroporation *[Methods. Enzymol*., *194*, 182 (1990)], spheroplast method [*Proc. Natl. Acad. Sci. USA*, 84, 1929 (1978)], lithium acetate method [*Journal of Bacteriology*, 153, 163 (1983)], etc. can be given.

As an expression vector in case of using an animal cell as a host cell, for example, pAGE107 [Japanese Patent Application Laid open No. 22,979/1991; *Cytotechnology*, 3, 133 (1990)], pAS3-3 (Japanese Patent Application Laid open No. 227,075/ 1990), pCDM8 [*Nature*, 329, 840 (1987)], pcDNAI/Amp (manufactured by Invitrogen), pREP4 (manufactured by Invitrogen), pAGE103 [*Journal of Biochemistry*, 101, 1307 (1987)], pAGE210, etc. are used.

As a promoter, any promoter can be used as far as it can be expressed in an animal cell, as examples of which IE (immediate early) gene promoter of cytomegalovirus (CMV), SV40 early promoter, metallothionein promoter, etc. can be given. In addition, human CMV IE gene enhancer may be used together with a promoter

As a host cell, Namalwa cell which is a human cell, COS cell which is a monkey cell, CHO cell which is a Chinese hamster cell, HBT5637 (Japanese Patent Application Laid open No. 299/1988), etc. can be enumerated.

As an introduction method of the recombinant vector, any method can be adopted as far as it is a method for introducing a DNA into an animal cell, as examples of which electroporation [*Cytotechnology*, 3, 133 (1990)], calcium phosphate method (Japanese Patent Application Laid open NO. 227,075/1990)], lipofection method [*Proc. Natl. Acad. Sci. USA* 84, 7413 (1987)], etc. can be given.

In case of using an insect cell as a host cell, a protein can be expressed according to the methods described, for example, in Baculovirus Expression Vectors, A Laboratory Manual; Current Protocols in Molecular Biology, Supplement 1 to 34; Bio/Technology, 6, 47 (1988); etc.

That is, after cotransfecting a recombinant gene-transfer vector and a baculovirus into an insect cell to obtain a recombinant virus in the supernatant of the insect cell culture, the insect cell is infected with the recombinant virus, and whereby a protein can be expressed.

As examples of the gene-transfer vector to be used in said method, pVL1392, pVL1393, pBlueBacIII (all manufactured by Invitrogen), etc. can be given.

As a baculovirus, a virus which infects an insect belonging to the *Noctuidae* family such as *Autographa californica* nuclear polyhedrosis virus and the like can be used.

As an insect cell, Sf9 and Sf21 [Baculovirus Expression Vectors, A Laboratory Manual, W.H. Freeman and Company, New York (1992)] which are ovarian cells of *Spodoptera frugiperda*, High5 (manufactured by Invitrogen) which is an ovarian cell of *Trichoplusia ni*, etc. can be used.

As a cotransfection method of the above recombinant gene-transfer vector and baculovirus into an insect cell, for example, calcium phosphate method (Japanese Patent Application Laid-open No. 227,075/1990), lipofection method [Proc. Natl. Acad. Sci. USA, 84, 7413 (1987)], etc. can be enumerated.

As an expression method of a gene, secretory production, fused protein expression, etc. can be carried out in accordance with the methods described in Molecular Cloning, Second Edition in addition to direct expression.

When the expression is carried out using yeast, an animal cell or an insect cell, a glycosyleted protein can be obtained.

A transformant obtained as above is cultured in a medium to make it produce and accumulate the present protein in the culture and the protein is harvested from said culture, and whereby the present protein can be produced. The method for culturing the present transformant in a medium can be carried out according to a generally known method which is used for the culture of a host.

As a medium for culturing a transformant obtained by using a prokaryote such as *Escherichia coli* or the like or an eukaryote such as yeast or the like as a host, either a natural medium or a synthetic medium may be used as far as it contains a carbon source, a nitrogen source, an inorganic salt, etc. which are assimilable for said organism and in which the culturing of the transformant can be carried out efficiently.

As a carbon source, any carbon source will do as far as it is assimilable for said organism. For example, carbohydrates such as glucose, fructose, sucrose, molasses containing these, starch or starch hydrolysate, etc.; organic acids such as acetic acid, propionic acid, etc.; alcohols such as ethanol, propanol, etc.; etc. can be used.

As a nitrogen source, ammonium salts of inorganic acids or organic acids such as ammonia, ammonium chloride, ammonium sulfate, ammonium acetate, ammonium phosphate etc., other nitrogen-contained compounds, peptone, meat extract, yeast extract, corn steep liquor, casein hydrolysate, soybean meal or soybean hydrolysate, various microbial cells obtained by fermentation and their digests, etc. can be used.

As inorganic salts, potassium dihydrogen phosphate, dipotassium hydrogen phosphate, magnesium phosphate, magnesium sulfate, sodium chloride, ferrous sulfate, manganese sulfate, copper sulfate, calcium carbonate, etc. can be used.

The culturing is carried out generally under aerobic conditions such as shake culture, deep aeration spinner culture or the like. The culture temperature is preferably 15 to 40°C, and the culturing time is generally 16 to 96 hours. During the culturing, the pH is kept at 3.0 to 9.0. The adjustment of the pH is carried out using an inorganic or an organic acid, an alkaline solution, urine, calcium carbonate, ammonia, etc.

Besides, if necessary, antibiotics such as ampicillin, tetracycline, etc. may be added to a medium during the culture.

In case of culturing a microorganism transformed with an expression vector using an inducible promoter, an inducer may be added to a medium, if necessary. For example, isopropyl- ß -D-thiogalactopyranosido and the like in case of culturing a microorganism transformed with an expression vector using *lac* promoter and indole acrylic acid and the like in case of culturing a microorganism transformed with an expression vector using *trp* promoter may be added to the medium.

As a medium for culturing a transformant obtained using an animal cell as a host cell, a generally used RPMI1640 medium, an Eagle's MEM, a medium prepared by adding bovine fetal serum, etc. to these media or the like can be used.

The culturing is carried out usually under such conditions as in the presence of 5% CO₂ and the like. The culturing temperature is preferably 35 to 37°C, and the culturing time is generally 3 to 7 days.

Besides, if necessary, antibiotics such as kanamycin, penicillin, etc. may be added to a medium during the incubation.

As a medium for culturing a transformant obtained using an insect cell as a host, a generally used TNM-FH medium (manufactured by Pharmingen, Inc.), Sf-900 II SFM medium (manufactured by Life Technologies, Inc.), ExCell400 and ExCell405 (both manufactured by JRH Biosciences, Inc.), etc. can be used.

The culturing temperature is preferably 25 to 30°C, and the culturing time is generally 1 to 4 days.

Besides, if necessary, antibiotics such as gentamycin, etc. may be added to a medium during the culturing.

In order to isolate and purify a protein expressed according to the above method from the culture medium of the above transformant, generally known methods for enzyme isolation and purification may be used.

For example, in case that the present protein is expressed in cells in the soluble form, the cells are collected by centrifugation after the completion of culturing, suspended in an aqueous buffer, disrupted by an ultrasonic disrupter, a French press, a Manton Gaulin homogenizer, a Dynomill or the like to thereby obtain a cell-free extract solution. From the supernatant obtained by centrifuging said cell-free extract solution, a purified preparation can be obtained by employing generally known methods for enzyme isolation and purification, that is, methods such as solvent extraction, salting out with ammonium sulfate or the like, desalting, precipitation with an organic solvent, anionic exchange chromatography using resins such as diethylaminoethyl (DEAE)-Sepharose, DIAION HPA-75 (manufactured by Mitsubishi Chemical Corporation) and the like, cationic exchange chromatography using resins such as S-Sepharose FF (manufactured by Pharmacia) and the like, hydrophobic chromatography using resins such as butyl Sepharose, phenyl Sepharose and the like, gel filtration using molecular sieves, affinity chromatography, electrophoresis such as chromatofocusing, isoelectric focusing and the like independently or in combination.

Besides, in case that said protein is expressed intracellularly as an inclusion body, said protein is recovered according to an generally known method from precipitated fractions obtained similarly by disrupting cells after collection and then centrifuging the disrupted cells, and subsequently said protein as an inclusion body is solubilized with a protein denaturant. After structuring said protein to have a correct structure by diluting said solubilized solution to a protein denaturant-free solution or a solution in which the concentration of the protein denaturant is so lowered as not to denature the protein or by dialyzing said solubilized solution, a purified preparation can be obtained according to the same isolation and purification method described above.

In case that the present protein or its derivative such as a glycosylated form or the like is extracellularly secreted, said protein or its derivative such as a glycosylated form or the like can be recovered from the culture supernatant. That is, said culture is treated according to the same method described above such as centrifugation and the like to obtain soluble fractions, from which a purified preparation can be obtained by employing the same isolation and purification method described above.

Besides, a protein expressed according to the above method can be produced also by chemical synthesis methods such as Fmoc method (fluorenylmethyloxycarbonyl method), tBoc method (t-butyloxycarbonyl method), etc. In addition, it is also possible to synthesize said protein by utilizing a peptide synthesizer available from Sowa Trading Co., Ltd. (manufactured by Advanced ChemTech, U.S.A.), Perkin-Elmer Japan (manufactured by Perkin-Elmer, U.S.A.), Pharmacia Biotech, K.K. (manufactured by Pharmacia Biotech, Sweden), Aloka Co., Ltd. (manufactured by Protein Technology Instrument, U.S.A), Kurabo Industries Ltd. (Synthecell-Vega, U.S.A.), Nippon PerSeptive Ltd. (PerSeptive, U.S.A.), Shimadzu Corporation or the like.

### 3. Preparation of Alzheimer's disease related Antibody

As the antibody of the present invention, any antibody such as a polyclonal antibody, a monoclonal antibody or the like will do as far as it can bind specifically to the above protein or polypeptide.

The polyclonal antibody can be prepared by separating and purifying sera obtained from an antigen-immunized animal. The monoclonal antibody can be prepared by fusing an antibody-producing cell obtained from an antigen-immunized animal with a myeloma cell to produce a hybridoma, culturing said hybridoma or inoculating the same to an animal to make the ascitic tumor and then separating and purifying said culture medium or said ascites.

The antigen can be prepared by purifying the protein of the present invention from various human cultured cells or by introducing a recombinant vector containing a DNA encoding a protein having the amino acid sequence represented by SEQ ID NO. 2 or a DNA encoding a protein having an amino acid sequence which includes substitution, deletion or addition of one or more amino acids of the amino acid sequence represented by SEQ ID NO. 2 and having apoptosis-suppressing activities into a host such as *Escherichia coli*, yeast, an animal cell, an insect cell or the like to make the host express the protein and then isolating and purifying the protein. In addition, the antigen can be prepared also by synthesizing a peptide having a partial sequence of the amino acid sequence represented by SEQ ID NO. 2 using an amino acid synthesizer.

As an immunization method, the antigen may be subcutaneously, intravenously or intraperitoneally administered to a non-human mammal such as a rabbit, a goat, a 3 to 20-week aged rat, mouse or hamster, or the like as it is. However, it is preferable that the antigen is administered either upon conjugating it to a highly antigenic carrier such as keyhole lympet hemocyanin, bovine serum albumin, bovine thyroglobulin or the like or together with an appropriate adjuvant such as Freund's complete adjuvant, aluminium hydroxide gel, pertussis vaccine or the like.

Administration of the antigen is carded out 3 to 10 times at 1 to 2 week intervals after the 1st administration. On the 3rd to 7th day after each administration, blood is collected from the venous plexus of ocular funds to examine whether or not said serum reacts with the antigen used for immunization by measuring antibody titer according to an enzyme immunoassay (Antibodies―A Laboratory Manual, Cold Spring Harbor Laboratory, 1988) or the like. A non-human mammal whose serum shows an enough antibody titer against the antigen used for immunization is served as a supply source of the serum or the antibody-producing cell.

The polyclonal antibody can be prepared by separating and purifying said serum.

The monoclonal antibody can be prepared by fusing an antibody-producing cell obtained from an antigen-immunized animal with a myeloma cell to produce a hybridoma, culturing said hybridoma or inoculating the same to an animal to make an ascitic tumor and then separating and purifying said culture medium or said ascites.

As the antibody-producing cell, a splenocyte cell, an antibody-producing cell in a lymph node or the peripheral blood, particularly a splenocyte is used suitably.

As the myeloma cell, a mouse-derived established cell line such as P3-X63Ag8-U1(P3-U1) [*Current Topics in Microbiology and Immunology*, 18, 1 - 7 (1978)], P3-NS1/1-Ag41(NS-1) strain [*European J. Immunology*, 6, 511 - 519 (1976)], SP2/0-Ag14(SP-2) stain [*Nature*, 276, 269 - 270 (1978)], P3-X63-Ag8653(653) [*J. Immunology*, 123, 1548 - 1550 (1979)], P3-X63-Ag8(X63) [*Nature*, 256, 495 - 497 (1975)] or the like as an 8-azaguanine-resistant mouse (originated from BALB/c) myeloma cell line is used suitably.

The hybridoma cell can be made according to the following procedures.

An antibody-producing cell and a myeloma cell are mixed, suspended in a HAT medium (a medium prepared by adding hypoxanthine, thymidine and aminoputerine to a normal medium) and ten cultured for 7 to 14 days. After culturing, a part of the culture supernatant is subjected to an enzyme immunoassay or the like to select a cell which is reactive to an antigen but unreactive to an antigen-free protein. Subsequently, the selected cell is cloned by limiting dilution, followed by enzyme immunoassay to select a clone which shows a stably high antibody titer as a monoclonal antibody-producing hybridoma cell.

The monoclonal antibody can be prepared by separating and purifying a culture medium obtained by culturing the hybridoma or ascites obtained by intraperitoneally inoculating the hybridoma cell to an animal to make the animal ascitic cancer.

As a method for separating and purifying a polyclonal antibody or a monoclonal antibody, treatment by employing methods such as centrifugation, ammonium sulfate precipitation, caprylic acid precipitation, chromatography using a DEAE-Sepharose column, an anion exchange column, a protein A or G column, a gel filtration column, etc., independently or in combination can be given.

### 4. Utilization of Human Nap1 DNA, Protein or Antibody

(1) The mRNA of human Nap1 gene of the present invention can be detected by using the DNA described in 1 above according to the Northern hybridization method (Molecular Cloning, Second Edition), the PCR method [PCR Protocols (1990)], the RT (reverse-transcribed) -PCR method, etc.
(2) Brain tissues of healthy people and those of the subjects are screened for the human Nap1 gene mRNA according to the method described in (1) above and ten the healthy people and the subjects are compared for the mRNA abundance to examine whether or not the abundance is decreased in the subjects, and whereby the diagnosis of Alzheimer's disease in the subjects can be performed.
   In the case of Northern hybridization method, the abundance of mRNA can be determined by measuring the quantity of the hybridized probe in dependence on the label of said probe, for example, the radioactivity in the case of ³²P label and the intensity of fluorescence in the case of a fluorescent label. In the case of RT-PCR method, it can be determined, for example, by staining an amplified fragment with a DNA-specific fluorescent stain such as ethidium bromide, SYBR Green I or the like and subsequently measuring the intensity of fluorescence.
(3) Using the DNA of the present invention, the chromosomal location of human Nap1 gene can be determined according to somatic cell hybrid method [*Annual Review of Genetics* 9, 407 (1975)] or *in situ* hybridization method.
   In the somatic cell hybrid method, the number of a chromosome where the human Nap1 gene exists is determined as follows. Firstly, the subculture of a hybrid cell, in which a human cell and a mouse or hamster established cell line are fused, brings a hybrid cell clone in which only 1 or 2 human chromosomes remain, the remaining chromosome(s) is (are) specified to prepare such a panel of a series of hybrid cell clones which covers 23 human chromosomes. Such a hybrid cell panel is commercially available from Bios Laboratories, Inc. or the like. A genome DNA is prepared from each cell in the panel, followed by carrying out PCR serving this genome DNA as a template and using a human Nap1-specific primer to thereby examine the amplification of a DNA fragment corresponding to the human Nap1 gene. Then, it can be judged that the human Nap1 gene located on the human chromosome contained in a cell where amplification took place.
   On the other hand, in the *in situ* hybridization method [*Annals of Human Genetics,* 45, 135 (1981); *Cell,* 52, 51 (1988)], first of all, the hybridization is carried out to a human chromosome specimen using the human Nap1 DNA of the present invention as a probe. Then, a signal of hybridization to the probe is detected to specify the location of the signal on the specimen, and whereby not only the chromosome where the human Nap1 gene located but also the physical location on said chromosome can be specified. The probe is labeled with a radioactive isotope ³H or biotin, and a signal can be detected by autoradiography in the case of ³H labeling and by avidin labeled with a fluorescent pigment FITC in the case of biotin labeling.
(4) The antisense oligonucleotide described in 1 above is introduced into a cell to repress the transcription or the translation of the Nap1 gene, and whereby apoptosis can be induced in the cell. The introduction of the antisense oligonucleotide can be carried out in the same manner as that of a recombinant vector. Besides, as methods for detecting the apoptosis of a cell, (i) microscopic observation, (ii) TUNEL method, (iii) DNA ladder detection method, (iv) quantitation of DNA fragmentation ratio, (v) cell size distribution measurement, etc. can be enumerated [Latest Apoptosis Experimentation Techniques, Youdo-sha (1995)].
   In microscopic observation, morphological cell changes characteristic for apoptosis, for example, condensation of a nucleus, condensation of a chromatin, atrophy of an organelle, formation of an apoptotic body, shrinkage of the whole cell, etc. are observed. The TUNEL method is a method for detecting the digested end of a DNA to be formed by apoptosis by labeling said end with biotin or digoxigenin using terminal deoxynucleotidyl transferase (TdT). The DNA ladder detection method is a method for detecting the fragmentation of a chromatin DNA resulted from apoptosis by extracting a DNA from a cell and electrophoresing the extracted DNA on an agarose gel, and whereby the fragmented DNA is detected in a ladder state. The quantitation of DNA fragmentation ratio is a method by which only a fragmented, low molecular weight DNA is extracted and said DNA is quartitated using diphenylamine. The cell size distribution measurement is a method by which the increase of small size cells caused by contraction or fragmentation of cells resulting from apoptosis is measured utilizing a particle size analyzer such as Coulter multisizer or the like. According to these methods, whether or not apoptosis takes place in a cell can be detected.
(5) By repressing the transcription of DNA or the translation of mRNA using the antisense oligonucleotide (RNA/DNA) described in 1 above [*Kagaku* (Chemistry), 46, 681 (1991); Bio/Technology, 9, 358 (1992)], it is possible to induce apoptosis and to utilize the same for the treatment of diseases caused by the participation of apoptosis suprression in the state of diseases.
   For example, the oligonucleotide is injectionally administered to a patient in the affected part or systemically together with an appropriate carrier having a high affinity for cell membranes such as liposome, cholesterol or the like in order to facilitate its incorporation into cells and is made to be incorporated into the patient's cells to induce apoptosis, and whereby diseases can be treated.
   As examples of the diseases the state of which the suppression of apoptosis participates in, cancers, autoimmune diseases such as systemic lupus erythematosus, rheumatoid arthritis, multiple sclerosis and the like, etc. can be given.
   In many cancer cells, mutations such as deletion and the like in genes such as p53 which is an apoptosis-inducing factor have been found [*British Journal of Cancer*, 68, 653 (1993)]. And, it is considered that, in such cells, apoptosis mechanism which, in normal tissues, excludes cells having abnormalities such as gene mutation and the like becomes unworkable to thereby advance the proliferation of cancer cells. In addition, there also is a report that the antisense oligo DNA of bc1-2 which works as an apoptosis-suppressing gene in many cells can inhibit the growth of human colon cancer in an animal experiment (NIKKEI'S DICTIONARY OF LATEST BIO TERMINOLOGY, Nikkei BP Inc., 1995).
   Therefore, the antisense oligonucleotide of the present invention can be utilized for the treatment of apoptosis-related cancers by suppressing the expression of the gene of the present invention using said antisense oligonucleotide to induce apoptosis.
   On the other hand, it is considered that autoimmune diseases outbreak because autoreactive T cells, which are generally excluded by apoptosis, are not excluded, and it has been known that a mouse possessing a mutated of Fas gene, which is regarded as an important apoptosis-inducing gene in this mechanism, or a mutant of a gene encoding a ligand of the Fas protein shows symptoms of autoimmune diseases because the expression of the Fas gene or Fas protein is repressed [*Nature,* 356, 317 (1992) and *Cell*, 75, 1169 (1993)].
   Therefore, autoimmune diseases can be treated by suppressing the expression of the gene of the present invention using the antisense oligonucleotide of the present invention to thereby induce apoptosis.
(6) Human Nap1 protein can be obtained using the DNA described in 1 according to the method described in 2.
(7) The protein described in 2 can be utilized for the treatment of Alzheimer's disease by administering the same to the brain of an Alzheimer's disease patient to suppress the apoptosis of neuronal cells. In addition, the suppression of cell apoptosis can be expected by the administration of this protein, so that it can be utilized for the treatment of diseases the state of which the induction of apoptosis participates in.
   A therapeutic composition for apoptosis-participating diseases which contains the protein of the present invention as an active ingredient can generally be systemically or focally administered parenterally. As a parenteral administration method, intravenous injection such as drip infusion or the like, intramuscular injection, intraperitoneal injection or subcutaneous injection can be selected, and it can be selected suitably in response to ages and symptoms of patients. The dosage of said composition differs in age, administration route and administration time and thus can be changed widely. In this case, the effective dose of the protein of the present invention and the effective dose to be administered as a composition with an appropriate diluent and a pharmacologically adoptable carrier are selected from the range of 10 *µ* g to 10mg/kg weight/time.
   As a dosage form to be applied to the case of parenterally administrating the protein of the present invention, generally a unit-dose ampoule or a multiple-dose container (a vial) which each contains additives such as a stabilizer, a buffer, a preservative, a tonicity agent, etc. is given.
   Furthermore, a therapeutic composition containing the protein of the present invention for apoptosis participating diseases may contain a pharmaceutically acceptable carrier and additives according to its administration route. As examples of such a carrier and additives, water, pharmaceuticalily acceptable organic solvents, collagen, polyvinyl alcohol, polyvinylpyrrolidone, sodium carboxymethylcellulose, sodium polyacrylate, sodium alginate, water soluble dextran, sodium carboxymethyl starch, pectin, methylcellulose, ethylcellulose, xanthane gum, gum arabic, casein, gelatin, agar, glycerin, polyethylene glycol, vaseline, paraffin, stearic acid, human serum albumin (HSA), mannitol, sorbitol, lactose, surfactants acceptable as pharmaceutical additives, etc. can be given. An additive to be used, though it is selected from the above suitably or in combination according to the dosage form of the present invention, is nowise restricted to these.
   As diseases to which the administration of the protein of the present invention is applied, that is, diseases the state of which the induction of apoptosis participates in, progressive neuro degenerative diseases, e.g., Parkinson's disease [*Journal of Neurological Sciences,* 137, 120 (1996)] and amyotrophic lateral sclerosis [*Neuropathology and Applied Neurobiology*, 21, 498 (1995)]; Huntington's chorea [*Experimental Neurology*, 133, 265 (1995); *Neuroreport*, 6, 1053 (1995) and *Journal of Neuroscience* 15, 3775 (1995)]; retinitis pigmentosa [Neuron, 11, 595 (1993) and Proc. Natl. Acad. Sci. USA 91, 974 (1994)]; glaucoma [*Investigative Ophthalmology and Visual Science* 36, 774 (1995) and *Journal of Glaucoma,* 5, 345 (1996)]; alcoholic hepatitis [*Journal of Hepatology*, 6, 137 (1988) and *Journal of Pathology*, 171, 73 (1993)]; etc. are given.
(8) An antibody can be produced using the protein described in 2 as an antigen according to the method described in 3.
(9) Using the antibody described in 3, the human Nap1 protein can be detected.
   Specifically, detection methods employing ELISA method and fluorescent antibody method using a microtiter, Western blot method, etc. can be enumerated.
(10) The antibody described in 3 can be utilized for immunological tissue staining of the brain tissues of subjects.
(11) Alzheimer's disease in a subject can be diagnosed by detecting human Nap1 protein derived from the brain tissue of a healthy person and that of a subject, measuring the quantities of said protein in the both brains and comparing the quantities to examine the decrease of said quantity in the subject.
(12) The antibody described in 3 can be utilized for the treatment of diseases the state of which the suppression of apoptosis participates in because it is expected to suppress the human Nap1 activity of cells to induce apoptosis by the administration of the antibody described in 3.

The dose and the dosage form are the same as aforementioned.

As examples of apoptosis-participating diseases, cancers; autoimmune diseases such as systemic lupus erythematosus, rheumatoid arthritis, multiple sclerosis, etc.; etc. can be given.

### BRIEF DESCRIPTION OF THE DRAWINGS

Fig. 1 is an electrophoretogram showing the result of RT-PCR.
Fig. 2 is a list showing the comparison of amino acid sequences between human Nap-1 and rat Nap-1.
Fig. 3 is an electrophoretogram showing the distribution of human Nap1 expression in various tissues investigated by Northern blotting.
Fig. 4 is an electrophoretogram showing the results of RT-PCR of Nap1 gene using a human neuronal cell line.
Fig. 5 are photographs showing the results of electrophoresis of cellular DNA after making an antisense PS-ODN be incorporated into the neuronal cell line SH-SY5Y.

### BEST MODE FOR CARRYING OUT THE INVENTION

Hereinafter, the present invention will be described further specifically by means of examples. However, the technical scope of the present invention is nowise limited to these examples.

### Example 1 Cloning of cDNA Fragment of Human Nap1 Gene

### (1) Preparation of the total RNA from the brain

From the cerebral cortex of 10 sporadic Alzheimer's disease patients and 3 non-Alzheimer's disease subject, the total RNAs were obtained using TRIzol reagent (manufactured by Life Technologies). Specific methods employed were in accordance with the instructions given in the manual of the reagent. These RNAs were treated with ribonuclease-free deoxyribonuclease (manufactured by Promega) to remove contaminating DNAs, extracted with phenol-chloroform, precipitated with ethanol and then dissolved in distilled water treated with diethyl pyrocarbonate (DEPC).

### (2) Differential display using the total RNA

According to the method of Ito et al. *[FEBS Letters*, 351, 231 (1994)], differential display was carried out. That is, 2.5 *µ* g each of the RNAs obtained in (1) were mixed with 50pmol of one anchor primer out of three primers of SEQ ID NOS. 4 to 6, followed by addition of DEPC-treated distilled water to give a total volume of 10 *µ* l. After heating at 70°C for 10 minutes, each RNA solution was chilled by immediately immersing in ice water. In this chilled solution, 10 *µ* l of 2× reverse transcriptase reaction buffer [40mM Tris-HCl (pH 8.4), 100mM KCl, 5mM MgCl₂, 2 *µ* l of 20mM dithiothreitol (DTT), 0.2mg/ml bovine serum albumin (BSA), 1mM dNTP (dATP, dGTP dCTP, dTTP)] containing 200 units of SUPERSCRIPT II RNase H Reverse Transcriptase (manufactured by Life Technologies) as a reverse transcriptase was mixed, reacted at 25°C for 10 minutes and at 42°C for 50 minutes to synthesize a cDNA, heated at 90°C for 5 minutes and then stopped the reaction. To this reaction solution, 80 *µ* l of TE buffer [10mM Tris-HCl (pH 8.0), 1mM EDTA] was added to dilute 5-fold.

To 2 *µ* l each of the synthesized cDNA, 2 *µ* l of 10× PCR buffer [100mM Tris-HCl (pH 8.8), 500mM KCl, 15mM MgCl₂, 1% Triton X-100, 1mM dNTP], 5pmol of an anchor primer (the same primer used in cDNA synthesis), 10pmol of an arbitrary primer (one out of 10 primers of SEQ ID NOS. 7 to 16), 1 unit of Gene Taq DNA polymerase (manufactured by Nippon Gene) were added and followed by addition of distilled water to give a total volume of 20 *µ* l, which was ten placed in a GeneAmp PCR System 9600 PCR apparatus (manufactured by Perkin Elmer).

PCR was carried out on condition that, after the reactions at 94°C, 37°C and 72°C for each 2 minutes, a cycle of reaction steps consisting of 94°C for 30 seconds, 55°C for 1 minute and 72°C for 1 minute was repeated 25 times and followed by the final reaction at 72°C for 5 minutes. Since 3 kinds of anchor primers and 10 kinds of arbitrary primers were used, 30 sets of samples were subjected to the PCR.

To 2 *µ* l each of reaction solutions after PCR, 2 *µ* l of formamide dye solution (98% formamide, 0.01% methyl violet, 10mM EDTA) was added. The mixture was heated at 90 °C for 2 minutes and then ice-cooled. The reaction solution was electrophoresed on a 6% acrylamide gel (20cm wide × 33cm high × 0.35mm thick) at 20mA for 2 hours using a Tris-Glycine buffer After fluorescently staining DNA with 0.01% SYBR Green I (manufactured by Molecular Probe), approx. 1,200 fragments amplified by PCR were detected and compared using Fluorimager 575 (manufactured by Molecular Dynamics). Out of the above fragments, a band showing remarkable reduction common throughout 10 cases of the brains of sporadic Alzheimer's disease patients in comparison with 3 cases of the brains of non-Alzheimer's disease subjects was selected. The amplification product which contained this band was obtained by the PCR using the primer of SEQ ID NO. 4 as an anchor primer and that of SEQ ID NO. 7 as an arbitrary primer

### (3) Cloning of selected amplified fragment band

The picture of the whole gel containing the selected band was printed out to have a actual-size enlargement, which was accurately superposed upon the gel to cut out the selected band from the gel. The remaining gel was scanned by a fluorimager to confirm that the band was cut out. After adding the gel cut out to 50 *µ* l of a PCR solution [1× PCR buffer containing an anchor primer (SEQ ID NO. 4) and an arbitrary primer (SEQ ID NO. 7)], 1 unit of a Gene Taq DNA polymerase was added thereto to carry out PCR, and whereby fragments contained in the gel was further amplified. PCR was carried out on condition that a cycle of reaction steps consisting of 94°C for 30 seconds, 55°C for 1 minute and 72°C for 1 minute was repeated 30 times.

The solution after reaction was precipitated with ethanol and then dissolved in 10 *µ* l of TE buffer

One *µ* l of the amplified fragment and 1 *µ* l of a PCR fragment cloning vector pT7Blue T-Vector (manufacture by Novagen; a vector prepared by digesting the *Eco*R V site of a plasmid T7Blue and adding one dT to the 3'-end of the both strand) were mixed, followed by ligating the amplified fragment into the plasmid using a DNA ligation kit Ver. 2 (manufactured by Takara Shuzo K.K.) according to the instructions given in the kit. *Escherichia coli* DH5 α (manufactured by Life Technologies) was transformed to obtain an ampicillin-resistant strain. Said transformant colony was pricked with a sterilized tooth pick and suspended in 25 *µ* l of PCR solution, followed by addition of 1 unit of Gene Taq DNA polymerase to carry out PCR under the same conditions as the reamplification of the amplified fragment. Two *µ* l of this solution and 2 *µ* l of PCR solution used when this amplified fragment was obtained in (2) were juxtaposed on the same gel to carry out the same electrophoresis and fluorescent staining as the differential display, followed by detecting an amplified fragment by a fluorimager. Because the fragment was amplified on quite the same position as the selected band observed in the PCR solution described in (2) according to PCR using a colony as a template, it was judged that said transformant contained the DNA of the selected band.

This transformant was named Clone 9-1, from which plasmid p9-1 was isolated.

### (4) Determination of the nucleotide sequence of amplified fragment

The nucleotide sequence of the amplified fragment contained in p9-1 was determined. The reaction for sequencing was carried out using SequiTherm Long-Read Cycle Sequencing Kit (manufactured by Epicentre Technologies), and the sequence was determined using ALF DNA Sequencer (manufactured by Pharmacia). The reagents and the methods employed were in accordance with the instructions given in the kit. The obtained nucleotide sequence was represented by SEQ ID NO. 3. Said nucleotide sequence comprised 265 nucleotides, which corresponded to the 3986th to the 4236th of the nucleotide sequences represented by SEQ ID NO. 1. Incidentally, the 1st to the 15th of the sequence represented by SEQ ID NO. 3 are derived from the arbitrary primer (SEQ ID NO. 7) and the 241st to the 265th of the sequence are derived from the anchor primer (SEQ ID NO. 4), so that the 1st to the 7th and the 259th to the 265th of the sequence represented by SEQ ID NO. 3 do not correspond to the base sequence represented by SEQ ID NO. 1.

### Example 2 Confirmation of Expression Specificity of Clone 9-1 Gene by RT-PCR

In addition to the total RNAs from the cerebral cortices of 10 sporadic Alzheimer's disease patients and 3 non-Alzheimer's disease subjects isolated in Example 1, the total RNAs were isolated from the cerebral cortices of 7 more non-Alzheimer's disease subjects in the same manner as in (1) of Example 1. From 1 *µ* l each of these total RNAs, a single-stranded cDNA was synthesized using SUPERSCRIPT II RNase H reverse transcriptase and a random hexamer primer (manufactured by Life Technologies). The specific reagents and methods employed were in accordance with the instructions given in the manual attached by Life Technologies.

The Alzheimer's disease patients were compared with the non-Alzheimer's disease subjects with respect to the abundance of mRNA of Clone 9-1 gene in the cerebral cortex. That is, using 2 *µ* l of the solution after reaction, PCR was carried out in 1× PCR buffer by adding thereto 10pmol of 5'-end sense primer (SEQ ID NO. 17), 10pmol of 3'-end antisense primer (SEQ ID NO. 18) and 1 unit of Gene Taq DNA polymerase. PCR was carried out on condition that, after the heating at 94°C for 3 minutes, a cycle of reaction steps consisting of 94°C for 30 seconds, 55°C for 1 minute and 72°C for 1 minute was repeated 28 times and followed by the final reaction at 72°C for 5 minutes.

After electrophoresing on a 1% agarose gel, the gel was stained with 0.01% SYBR Green I to detect bands for the amplified fragment (corresponding to the 27th to the 211th of the nucleotide sequence represented by SEQ ID NO. 3 and the 4005th to the 4189th of the nucleotide sequence represented by SEQ ID NO. 1).

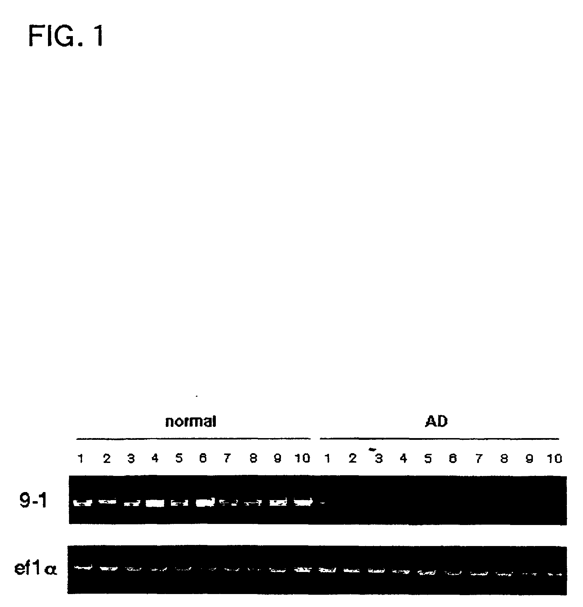

As a consequence, the results shown in Fig. 1 were obtained.

Fig. 1 is electrophoretograms showing the results of RT-PCR using cerebral cortex RNAs of Alzheimer's disease patients and non-Alzheimer's disease subjects with respect to Clone 9-1 cDNA (=human Nap-1 gene cDNA), wherein AD 1 to 10 of the lane indicates the cerebral cortex RNAs of Alzheimer's disease patients and normal 1 to 10 indicates those of non-Alzheimer's disease subjects, and 9-1 indicates the results in the case of having used a Nap-1 gene-specific primer to the foregoing cerebral cortex RNAs and ef1 α indicates the results in the case of having used a primer specific to an elongation factor 1 α gene used as a control to said RNAs.

From the results shown in Fig. 1, the quantity of amplified fragment is decreased in all of 10 sporadic Alzheimer's disease patients as compared with 10 non-Alzheimer's disease subjects as controls, from which it is confirmed that the abundance of mRNA of the Clone 9-1 gene is decreased in the cerebral cortex of Alzheimer's disease patients.

Incidentally, the unchangeness of abundance of mRNA itself was confirmed by carrying out the same RT-PCR with respect to an elongation factor 1 α (ef1 α) gene which is a housekeeping gene using ef1 α -specific primers represented by SEQ ID NOS. 19 and 20. Said PCR was carried out on condition that, after the heating at 94°C for 3 minutes, a cycle of reaction steps consisting of 94°C for 30 seconds, 58°C for 1 minute and 72°C for 2 minutes was repeated 25 times and followed by the final reaction at 72°C for 5 minutes.

### Example 3 Cloning of Full-length cDNA of Clone 9-1

### (1) Isolation of cDNA clones from cDNA library

Clones of cDNA was isolated from a human cerebral cortex cDNA library (manufactured by CLONETECH) by plaque hybridization. A nylon membrane filter (Hybond N+; manufactured by Amersham) on which 2×10⁵ plaques were blotted was sealed in a hybridization solution [6×SSC, 10×Denhart's solution (0.2% BSA, 0.2% Ficoll, 0.2% polyvinyl pyrrolidone), 1% (w/v) SDS, 200 *µ* g/ml of salmon sperm DNA denatured (heated for 5 minutes in boiled water and the rapidly cooled in ice) after ultrasonic treatment] and prehybridized at 60°C for 2 hours.

Probes were made as follows. That is, PCR was carried out in 1× PCR buffer in the same manner as in Example 1 using the primers of SEQ ID NOS. 21 and 18 and serving the plasmid DNA of Clone 9-1 as a template, thereby amplifying the cDNA moiety corresponding to the 26th to the 211th of the nucleotide sequence represented by SEQ ID NO. 3. PCR was carried out on condition that a cycle of reaction steps consisting of 94°C for 20 seconds, 58°C for 30 seconds and 72°C for 1 minute was repeated 30 times.

The solution after PCR was electrophoresed on a 6% acrylamide gel, the band for the amplified fragment was cut out from the gel, and PCR was repeated under the same conditions serving this cut gel as a template to reamplify the fragment. The reaction solution was precipitated with ethanol, followed by dissolving in 10 *µ* l of distilled water To 1 *µ* l of this solution, 5 *µ* l of 10× buffer [100mM Tris-HCl (pH 8.8), 500mM KCl, 1% Triton X-100], each 1 *µ* l of 10pmol primers (SEQ ID NOS. 22 and 23), 1 *µ* l of 4mM (dATP dGTP, dTTP), 10 *µ* l of dCTP (manufactured by NEN) and 1 *µ* l of Gene Taq were added to carry out PCR again under the same conditions, thereby making a ³²P-labeled probe.

From the reaction solution, a probe was purified using a quick spin column G-50 (DNA) (manufactured by Boehringer Mannheim). This probe was added to the hybridization solution, and the filter was sealed therein and hybridized overnight at 60°C. After washing the filter with 0.2×SSC containing 0.2% (w/v) SDS at 60°C for 30 minutes, an imaging plate (manufactured by Fuji Photo Film Co., Ltd.) was exposed and positive plaques were detected using Bioimaging Analyzer BAS2000A (manufactured by Fuji Photo Film Co., Ltd.) and then isolated. Since this cDNA library uses λ ZAP II as a vector, *in* *vivo* excision was carried out using the isolated positive plaques, and whereby an *Escherichia coli* transformant (*Escherichia coli* SOLR/p9-1-6) containing a cDNA clone p9-1-6 whose vector moiety was changed from λ ZAP II to plasmid pBluescript II was obtained. Specific methods for the *in vivo* excision employed were in accordance with those given in the manual of Stratagene.

Incidentally, the *Escherichia coli* transformant *Escherichia coli* SOLR/p9-1-6 was internationally deposited as FERM BP-6201 in National Institute of Bioscience & Human-technology, Agency of Industrial Science & Technology (Higashi 1-1-3, Tsukuba City, Ibaraki, Japan) on December 10, 1997 under the Budapest Treaty.

### (2) Analysis of nucleotide sequence of cDNA contained in p9-1-6

The nucleotide sequence of the p9-1-6 cDNA was determined in the same manner as in (4) of Example 1. As a result of it, a 4071-bp nucleotide sequence corresponding to the 166th to the 4236th of SEQ ID NO. 1 could be determined, but the nucleotide sequence from here to the 5'-end could not be determined.

### (3) Isolation of cDNA clone by 5'-RACE

In order to clarify the nucleotide sequence of the whole cDNA, 5'-RACE was carried out. With respect to human brain poly(A)⁺RNA (manufactured by Clonetech), approx. 0.3-kb DNA fragment considered to contain cDNA on the 5'-end side from the cDNA moiety corresponding to the primer sequence (corresponding to the 314th to the 338th of the nucleotide sequence represented by SEQ ID NO. 1) was amplified by using the cDNA-specific primer described in SEQ ID NO. 22 and 5'-RACE System as a 5'-RACE kit manufactured by Life Technologies.

Specific methods of the 5'-RACE employed were in accordance with the instructions given in the kit's manual. This DNA fragment was cloned into a vector pT7Blue T-Vector in the same manner as in (3) of Example 1 to obtain pRCOM.

A pRCOM-containing *Escherichia coli* transformant *Escherichia coli* DH5 α /pRCOM was internationally deposited as FERM BP-6202 in National Institute of Bioscience & Human-technology, Agency of Industrial Science & Technology (Higashi 1-1-3, Tsukuba City, Ibaraki, Japan) on December 10, 1997 under the Budapest Treaty.

### (4) Sequencing of cDNA

The nucleotide sequence of cDNA of the clone pRCOM obtained by 5'-RACE was determined in the same manner as described in (5) of Example 1, and this nucleotide sequence and the nucleotide sequence of the p9-1-6 cDNA determined in (2) of Example 4 were ligated to obtain a nucleotide sequence considered to be the full-length cDNA of 9-1. This nucleotide sequence consists of 4236 base pairs, in which an open reading frame (ORF) consisting of 1128 amino acids existed. This amino acid sequence was represented by SEQ ID NO. 2.

As a result of comparing this nucleotide sequence with the nucleotide sequence data base GenBank using a homology searching program BLAST, a nucleotide sequence in identical with said nucleotide sequence was absent in the known nucleotide sequences except some, for example, those of Access Nos. AA307472, R58000, N44074, etc. were found in EST, and thus this nucleotide sequence was a novel one. Incidentally, ESTs which have identical sequence with 9 - 1 were considered to be of nonfull-length cDNAs because the identical sequence occurred from the midway of the ORE. In addition, it was rat Nap1 gene that showed the highest homology among the known nucleotide sequences.

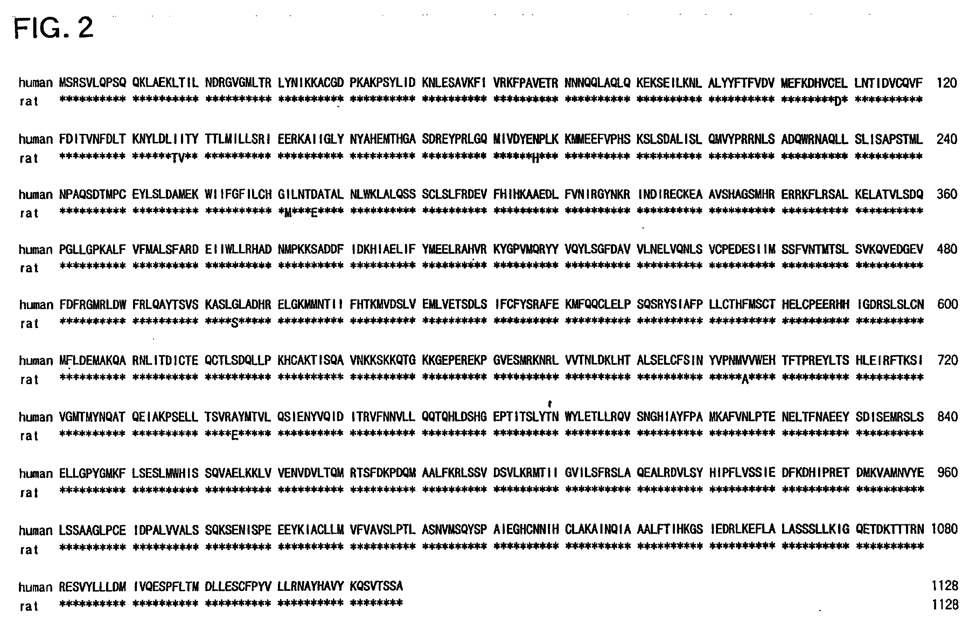

The results were shown in Fig. 2. Fig. 2 is a list showing the comparison of the amino acid sequences of human Nap1 and rat Nap1. By single letter notation, the amino acid sequence of the human Nap1 was shown in the raw marked as 'human' and that of the rat Nap1 in the raw marked as 'rat', provided that, in the case of the rat Nap1, the same amino acids as in the case of the human Nap1 were marked by asterisk and different amino acids alone were noted by single letters. The numbers given on the right ends show the number of amino acids.

As shown in Fig. 2, the amino acid sequence of ORF has 99.2% homology with that of rat Nap1 ORF, and the number of its amino acids was also 1128 which was quite the same. Therefore, this cDNA was considered to be the cDNA of the human Nap1 gene.

### Example 4 Determination of Chromosomal location of Human Nap 1 Gene

The clomosomal location of the human Nap1 gene was determined according to the method of Ruddle et al. [*Annual Review of Genetics,* 9, 407 (1975)] by using a human-rodent hybrid cell panel (manufactured by BIOS Laboratories).

That is, genome DNAs were purified from hybrid cells respectively corresponding to human chromosomes. To 100ng each of said chromosomal DNAs, PCR was carried out using a sense primer (SEQ ID NO. 23) and an antisense primer (SEQ ID NO. 18) amplifying the 3'-end region, which was not preserved between human and rat Nap1 genes (corresponding to the 3837th to the 4189th of the nucleotide sequence represented by SEQ ID NO. 1) out of the nucleotide sequence of the human Nap1 gene, and an EX Taq DNA polymerase (Manufactured by Takara Shuzo K.K.).

PCR was carried out on condition that the reaction was firstly conducted at 94°C for 1 minute, then a cycle of reaction steps consisting of 94°C for 1 minute, 61°C for 1 minute and 72°C for 1.5 minutes was repeated 30 times and followed by the reaction at 72°C for 5 minutes. As a result, amplification was observed only in a DNA containing human chromosome 2, and thus the human Nap1 gene was considered to exist in the chromosome 2.

In order to determine the chromosomal location of the Nap1 gene in further detail, fluorescence *in situ* hybridization (FISH) was carried out based on literature [*Genomics,* 17, 153 (1993)]. That is, a human metaphase chromosome was prepared from lymphocytes of a normal male according to thymidine synchronization, and an R band stained specimen was made according to bromodeoxyuridine releasing method. Before hybridization, the chromosome was stained with Hoechst 33258 and then irradiated with UV light. Approximately 4.2kb of hNAP1 cDNA was labeled with biotin according to nick translation using biotin-16-dUTP for a probe. The biotin-labeled probe was added to a hybridization solution[50% formamide, 10% dextran sulfate (manufactured by Sigma), 2×SSC, 2 *µ* g/ *µ* l of salmon sperm DNA denatured after sonication, 2 *µ* g/ *µ* l of *Escherichia coli* tRNA] to give a final concentration of 25 ng/ *µ* l, thereby hybridizing to the denatured chromosome specimen.

According to the method described in the literature [Proc. Natl. Acad. Sci. USA 83 2934 (1986)], the hybrid was reacted wit FITC-labeled avidin (manufactured by Boehringer Mannheim) and further with biotin-labeled anti-avidin D (manufactured by Vector) in order to amplify the fluorescence, followed by reacting with FITC-labeled avidin again to detect the hybridized site. The chromosome was stained with 1 *µ* g/ *µ* l propidium iodide.

As a result, it was found that the chromosomal position of the present gene was q-32 of the long arm of the 2nd chromosome and that it existed in 2q32.1 - 2q32.2.

### Example 5 Detection of Expression of Human Nap1 mRNA in Various Tissues

### (1) Northern blotting of human Nap1 gene in various human tissues

In order to examine the expression of Nap1 gene in various human tissues, the expression of Nap1 mRNA in various human tissues was detected by Northern blotting using Clonetech's Filter MTN (Multiple Tissue Northern) Blots on which mRNAs of various human tissues are blotted. In the Human MTN Blot used, 2 *µ* g each of mRNAs of heart, brain, placenta, lung, liver, skeletal muscle, kidney and pancreas; in the Human NTN Blot II used, 2 *µ* g each of mRNAs of spleen, thymus, prostate gland, testis, ovary, small intestine, large intestine and peripheral blood leukocyte; and in the Human Brain MTN Blot III, 2 *µ* g each of mRNAs of cerebellar tonsil, caudate nucleus, corpus callosum, hippocampus, hypothalamus (the whole brain), substantia nigra, subthalamus (nucleus subthalamicus) and thalamus are blotted.

In order to use the Clone 9-1 cDNA fragment obtained in Example 1 as a probe, said cDNA fragment was labeled with ³²P using [α-³²P]dCTP and a Prime-It II labeling kit (manufactured by Stratagene). The specific reagents and methods for labeling employed were in accordance with the instructions given in the kit.

A filter was immersed and sealed in a hybridization solution [6×SSC, 10×Denhart's solution, 1% SDS, 200 *µ* g/ml of salmon sperm DNA denatured (heated for 5 minutes in boiled water and the rapidly cooled in ice) after ultrasonic treatment] and prehybridized at 60°C for 2 hours. Next, the probe was heated for 5 minutes in boiled water, denatured by rapidly cooling in ice and added to the hybridization solution, and the filter was immersed and sealed in the solution and hybridized overnight at 60°C.

The filter was taken out from the solution and washed by shaking in 0.2×SSC containing 0.2% SDS at 60°C for 30 minutes. The filter was superposed upon a imaging plate (manufactured by Fuji Photo Film Co., Ltd.) to carry out autoradiography, followed by analysis using Bioimaging Analyzer BAS2000A (manufactured by Fuji Photo Film Co., Ltd.).

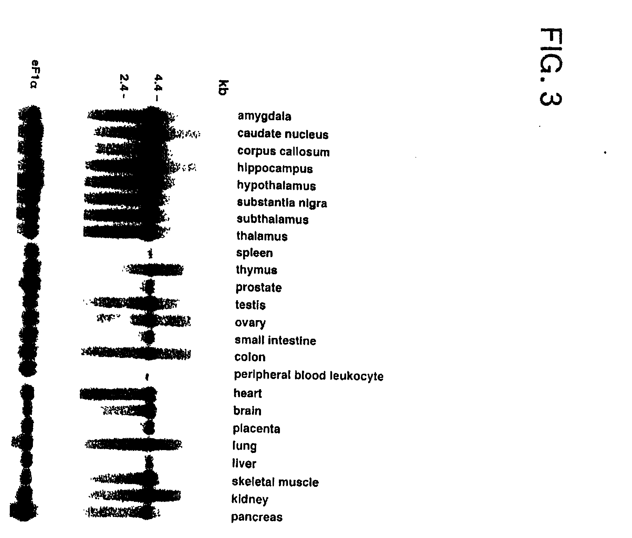

The results are shown in Fig. 3. In Fig. 3, the respective bands show, from the left, the results of Northern Blotting in cerebellar tonsil, caudate nucleus, corpus callosum, hippocampus, hypothalamus (the whole brain), substantia nigra, subthalamus (nucleus subthalamicus), thalamus, spleen, thymus, prostate gland, testis, ovary, small intestine, large intestine, peripheral blood leukocyte, heart, brain, placenta, lung, liver, skeletal muscle, kidney and pancreas. The arrow indicates the position of human Nap 1 mRNA. The bands for ef1 α shown beneath the respective bands are the results of Northern blotting of the elongation factor 1 α gene as a control.

As shown in Fig. 3, bands corresponding to 4.2-kb mRNA were detected in all the tissues excluding the peripheral blood leukocyte as to the human Nap1 gene. Therefore, it was found that the human Nap1 gene was a gene which is expressed ubiquitously in each tissue. Among the tissues, heart and skeletal muscle exhibited high expression. In addition, in respective tissues of the brain, it was expressed ubiquitously in all of the tissues.

### (2) RT-PCR of Nap1 gene in human neuronal cell line

In which of a neuronal cell and a glial cell expression will be observed mainly in brain was examined by RT-PCR of cell lines. SH-SY5Y [donated by Dr Shinichi KOUSAKA of National Institute of Neurological Science; *Cancer Research,* 33, 2643 (1973)] and LA-N-5 [donated by Dr. Takeshi YAMADA of Kyushu University; *Experimental Cell Research*, 148, 21 (1983)] as neuroblastoma cell lines and U-251 [donated by Dr Takeshi YAMADA of Kyushu University; *Journal of Neuropathology & Experimental Neurology,* 40, 390 (1981)] as a glioma cell line were respectively cultured in DMEMs (Dulbecco's modified Eagle's medium) each containing 10% bovine fetal serum, 100 units/ml penicillin and 100 mg/ml Streptomycin at 37°C in a 5% CO₂ incubator The total RNAs were isolated from the respective cells and subjected to RT-PCR in the same manner as in Example 2.

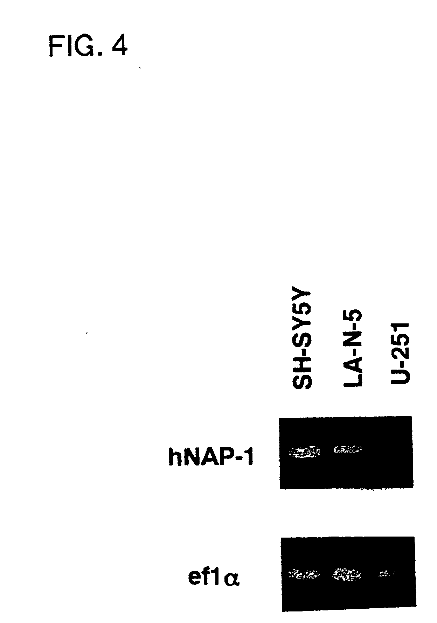

The results are shown in Fig. 4. In Fig. 4, SH-SY5Y, LA-N-5 and U-251 described on the left indicate the aforementioned respective cells, hNAP-1 indicates the results of having used a human Nap1 gene-specific primer and ef1 α indicates the results of having used a primer specific to the elongation factor 1 α gene used as a control.

As shown in Fig. 4, the expression of the Nap1 gene was detected in SH-SY5Y and LA-N-5 as neuroblastoma cell lines but not detected in U-251 as a glioma cell lines. Therefore, it was considered that the gene of the present invention was expressed mainly in a neuronal cell rather than in a glial cell.

### (3) In situ hybridization of human Nap1 gene in brain

In order to confirm that the gene of the present invention is expressed in the brain (mainly in nerve cells), *in situ* hybridization was carried out in human cerebral tissues.

Human cerebral tissues to be served as specimens are tissues of spinal cord, cerebellum, mesencephalon, basal ganglia, frontal cerebral cortex region and hippocampus, which were used by fixing with formalin after death and then embedding in paraffin.

First of all, PCR was carried out using a cDNA clone as a template and primers of SEQ ID NOS. 24 and 25 on condition that a cycle of reaction steps consisting of 94°C for 1 minute, 60°C for 1 minute and 72°C for 2 minute was repeated 30 times, thereby amplifying the moiety corresponding to the 314th to 1051st of SEQ ID NO. 1. Then, the amplified fragment was cloned into the *Eco*RV site of a vector pBluescript SK(-) (manufactured by Stratagene).

Serving this plasmid DNA as a template and using RNA Transcription Kit (manufactured by Stratagene), transcription was carried out *in vitro* by adding [³⁵S]UTP α S to the reaction solution, thereby making a ³⁵S-labeled single-stranded RNA probe. At this time, an antisense probe was made by using T7RNA polymerase after digesting the plasmid with the restriction endonuclease *Eco*RI (manufactured by Life Technologies) and a sense probe was made by using T3RNA polymerase after digesting the plasmid with *Hind*III (Life Technologies).

The specific reagents and methods for the reaction employed were in accordance with the instructions attached to the kit. The *in situ* hybridization was carried out by somewhat modifying the method given in the literature [*Neuroscience Letters,* 194, 205 (1995)]. That is, the paraffin-embedded specimen was cut into 8 *µ* m thick sections and placed on silane-coated slide glasses. Incidentally, these procedures were so carried out that ribonuclease might not adhere to the specimen. After removing paraffin, the sections were immersed in 10mM PBS, reacted at 37°C for 10 minutes in 10 *µ g*/ml of proteinase K and then immersed in 0.1M triethanolamine containing 0-25% (v/v) acetic anhydride for 10 minutes. The section on a formamide paper was covered with 200 *µ* l of hybridization solution containing approx. 1×10⁶ cpm of RNA probe, kept at 65°C for 18 hours, thereby hybridizing to the probe. The section was washed in 2×SSC at 65°C for 30 minutes and then in a highly stringent wash (50% formamide, 2×SSC, 0.1M DTT) at 65°C for 30 minutes. The section was treated in 1 *µ* g/ml of ribonuclease A at 37°C for 30 minutes, air-dried, closely adhered to an autoradiographic film b-max (manufactured by Amersham) and then exposed for 48 hours. Next the section was taken out, immersed in an autoradiographic emulsion [50% (w/v) NR-2 (manufactured by Konica Corporation) distilled water solution], airdried and then exposed at 4°C for 28 days.

As a result, few signals were observed in the case of the sense probe, while high expression was observed in cerebellum, hippocampus and cerebral tonsil as were tissues in which neuronal cell crowded in high density in the case of the antisense probe. On the other hand, white matter where glial cells exist abundantly showed very low expression. In further examination at a microscopic level, high expression was observed in the granule all layer of cerebellum, Purkinje cell of cerebellum, neuronal cell of hippocampus, dentate gyrus, amygdaloid nucleus and cerebral cortex, and the expression was confirmed mainly in nerve cell rather than in glial cells. In addition, the same detection using this antisense probe could be carried out in regard to young matured rat's brain which had been embedded with paraffin after fixation. The fixation of the brain was carried out by anesthetizing a rat and then perfusing its brain with a 4% paraformaldehyde/10mM phosphate buffer.

### Example 6 Acceleration of Apoptosis by Antisense DNA of Human Nap1 Gene

### (1) Acceleration of cell death of SH-SY5Y cell by antisense DNA

The SH-SY5Y neuroblastoma cell line was cultured in the same manner as in (2) of Example 5. Human Nap1 antisense phosphorothioate oligodeoxynucleotide (PS-ODN) having a nucleotide sequence represented by SEQ ID NO. 26 and human Nap1 sense PS-ODN having a nucleotide sequence represented by SEQ ID NO. 27 as a control were synthesized (manufactured by Toagosei Chemical Industry Co., Ltd.). Each was mixed with 4 *µ* l of Lipofect Amine reagent (manufactured by Life Technologies), added to 200 *µ* l of DMEM and then incubated at room temperature for 30 minutes. Then, 800 *µ* l of DMEM was added to the incubate, and the resulting mixture was added to the culturing medium of SH-SY5Y cells so as to give a final PS-ODN concentration of 4.5 *µ* M*.* Further, the culturing was continued for 12 hours to make the PS-ODN be incorporated in the SH-SY5Y cells. The medium was exchanged for a medium free from PS-ODN, and the culturing was continued further. After 48 hours of medium exchange, many of the cells in which the antisense PS-ODN was incorporated in were changed to have a round cell shape and floated up in the medium, in which a large number of dead cells were observed. On the other hand, absolutely no change was observed in the control cells in which the sense PS-ODN was incorporated.

### (2) Acceleration of SH-SY5Y cell apoptosis by antisense DNA

In the same manner as in (1) above, cells were cultured for 12 hours upon adding thereto antisense PS-ODN or sense PS-ODN, thereby making the cells incorporate PS-ODN. After exchanging the medium, the cells were recovered by centrifugation at 1,000g for 5 minutes by every 12 hours in 60 hours. The recovered cells were lysed by adding a cytolysis buffer [50mM Tris-HCl (pH 8.0), 10mM EDTA, 0.3% Triton X-100] thereto and stationarily leaving on ice for 30 minutes. The lysed cells were fractionated into chromatin fractions (precipitate) and DNA fragment fractions (supernatant) by further centrifuging at 27,000g for 20 minutes.

Proteinase K was added to the supernatant at a concentration of 0.5 mg/ml and reacted at 50°C for 1 hour. Ribonuclease A was added to the reaction mixture to give a concentration of 0.4 mg/ml and further reacted at 50°C for 1 hour. Extraction was carried out twice with phenol-chloroform and then precipitated with ethanol, thereby purifying DNA. This DNA was lysed in a TE buffer and electrophoresed on a 1.5% agarose gel. As electrophoresis buffers, 40mM Tris-acetate and 1mM EDTA (pH 7.2) were used. After electrophoresis, DNA was stained with SYBR Green I and subjected to the detection of bands by a UV transilluminator.

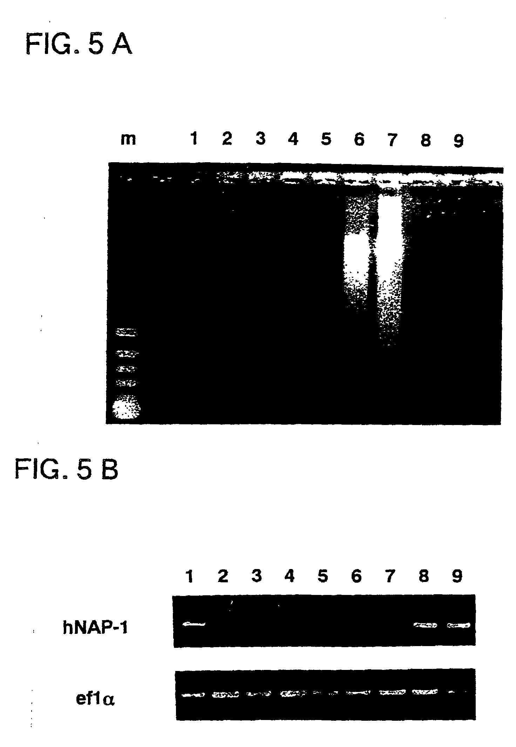

The results were shown in Fig. 5. Fig. 5A is an electrophoretogram showing the results of electrophoresis of cell DNA after making the SH-SY5Y neuronal cell line incorporate the antisense PS-ODN (SEQ ID NO. 26). From the left lane of this figure, (m) indicates a DNA size marker (φX174DNA/*Hae*III digestion); (1) indicates SH-SY5Y DNA in case of adding neither reagent nor PS-ODN; (2), (3), (4), (5), (6) and (7) respectively indicate SH-SY5Y DNA after 0 hour, 12 hours, 24 hours, 36 hours, 48 hours and 60 hours from the exchange of medium upon making the cells incorporate the antisense PS-ODN; (8) indicates SH-SY5Y DNA after 60 hours from the exchange of medium upon making the cells incorporate the sense PS-ODN (SEQ ID NO. 27); and (9) indicates SH-SY5Y DNA after 60 hours from the exchange of the medium upon adding a Lipofect Amine reagent alone.

Fig. 5B is an electrophoretogram showing the result of RT-PCR of human Nap1 gene after making the SH-SY5Y neuronal cell line incorporate the antisense PS-ODN (SEQ ID NO. 26). The culturing conditions for the cells in respective lanes are as same as those of Fig. 5A. In Fig. 5B, hNAP 1 shows the result in case of using a human Nap1 gene-specific primer and ef1 α shows the result in case of using a primer specific to elongation factor 1 α gene used as a control.

As shown in Fig. 5, DNA ladders characteristic to cells undergone apoptosis were observed in the supernatant of the cells after 48 hours and 60 hours from the exchange of medium upon making the cells incorporate the antisense PS-ODN. This DNA ladder was not observed in the cells after 60 hours from the addition of sense PS-ODN or a Lipofect Amine reagent alone. In addition, when cells made to incorporate antisense PS-ODN were recovered chronologically in the same manner described above and subjected to RT-PCR to human Nap1 gene in the same manner as in Example 2, it was confirmed that the expression of human Nap1 gene mRNA was lowered immediately after 12-hour culturing of the cells upon having added the antisense PS-ODN to make the cells incorporate the same (Fig. 5B).

Besides, with respect to the human Nap1 antisense PS-ODN represented by SEQ ID NO. 28 (manufactured by Toagosei Chemical Industry Co., Ltd.) corresponding to another site of the Nap1 gene and the control sense PS-ODN represented by SEQ ID NO. 29 (Toagosei Chemical Industry Co., Ltd.), DNAs of the SH-SY5Y cells, in which the above nucleotides were incorporated, after 60 hours from the change of media were electrophoretically examined similarly. As a result, DNA ladders characteristic to cells undergone apoptosis were observed in the supernatant of the cells in which antisense PS-ODN had been incorporated, while no DNA ladder was observed in the sense PS-ODN, either.

Therefore, it was found that the abundance of Nap1 gene was lowered to cause apoptosis of the SH-SY5Y cells by making the cells incorporate the antisense PS-ODN. From this, it is considered that the Nap gene suppress the apoptosis of cells.

Besides, the detection of apoptosis by *in situ* end label assay [*Journal of Cellular Biology*, 119, 493 (1992)] was also carried out. The SH-SY5Y cells were sprayed on a cover glass and cultured for 48 hours in the same medium as that of (3) of Example 5. After adding the antisense PS-ODN (SEQ ID NO. 26) or the control sense PS-ODN (SEQ ID NO. 27) to the medium, the medium was incubated for 12 hours to make the cells incorporate the PS-ODN, exchanged for a medium free from PS-ODN and further incubated for 24 hours. With respect to this cell, the detection of apoptosis was carried out using TACS In situ Apoptosis Detection Kit (manufactured by Trevegen). The specific reagents and methods employed were in accordance with the instructions of the kit. DNAs in the nucleosome digested by apoptosis were end-specifically labeled with Biotin, bound with peroxidase-labeled streptoavidin and then reacted using TBL as a peroxidase chromogenic reagent. As a result, deep blue was produced and apoptosis was detected in the cells in which the antisense PS-ODN had been incorporated, while apoptosis was not detected in the cells in which the control sense PS-ODN was incorporated.

### (3) Acceleration of apoptosis by antisense DNAs in

### LA-N-5 cells and retinoic acid-treated SH-SY5Y cells

Whether or not Nap1 gene antisense DNA causes apoptosis even in neuronal cells which are more differentiated than the SH-SY5Y cells was examined. That is, the SH-SY5Y cells cultured by adding thereto 10 *µ* M all trans retinoic acid (RA) (manufactured by Sigma) and the LA-N-5 neuroblastma cell line which is considered to be a little more differentiated than the SH-SY5Y cell were subjected to an experiment for making them incorporate the antisense PS-ODN represented by SEQ ID NO. 26 similarly to (2) above. Incidentally, it is said that the SH-SY5Y cell so differentiates as to have properties of a matured nerve cell such that, by the treatment with 10 *µ* M RA, it elongates neurites, it discontinues proliferation, its cell membrane generates an action potential and so

Also in the case of the 10 *µ* M RA-treated SH-SY5Y cells and the LA-N-5 cells, DNA ladders characteristic to cells undergone apoptosis were observed in the cells in which the antisense PS-ODN had been incorporated, but were not observed in the cells in which the sense PS-ODN had been incorporated. Therefore, it was confirmed that the Nap1 gene antisense DNA caused apoptosis even in a more differentiated neurocyte.

Besides, in the same manner described in (2) above, the detection of apoptosis by *in situ* end label assay was carried out in regard to LA-N-5 cells. As a result, apoptosis was detected in the antisense PS-ODN but not in the control sense PS-ODN similarly.

All of the publications, patents and patent applications cited in this specification shall be incorporated as they are in this application as references.

### INDUSTRIAL APPLICABILITY

According to the present invention, a human Nap1 protein, a DNA encoding said protein, a process for producing said protein, an antibody recognizing said protein and the uses of said protein are provided.

By the use of an novel gene to be obtained according to the present invention, the treatment and the diagnosis of Alzheimer's disease become possible.

### Free Text of Sequence Listing

SEQ ID NO. 4: Synthetic DNA
SEQ ID NO. 5: Synthetic DNA
SEQ ID NO. 6: Synthetic DNA
SEQ ID NO. 7: Synthetic DNA
SEQ ID NO. 8: Synthetic DNA
SEQ ID NO. 9: Synthetic DNA
SEQ ID NO. 10: Synthetic DNA
SEQ ID NO. 11: Synthetic DNA
SEQ ID NO. 12: Synthetic DNA
SEQ ID NO. 13: Synthetic DNA
SEQ ID NO. 14: Synthetic DNA
SEQ ID NO. 15: Synthetic DNA
SEQ ID NO. 16: Synthetic DNA
SEQ ID NO. 17: Synthetic DNA
SEQ ID NO. 18: Synthetic DNA
SEQ ID NO. 19: Synthetic DNA
SEQ ID NO. 20: Synthetic DNA
SEQ ID NO. 21: Synthetic DNA
SEQ ID NO. 22: Synthetic DNA
SEQ ID NO. 23: Synthetic DNA
SEQ ID NO. 24: Synthetic DNA
SEQ ID NO. 25: Synthetic DNA
SEQ ID NO. 26: Synthetic DNA
SEQ ID NO. 27: Synthetic DNA
SEQ ID NO. 28: Synthetic DNA
SEQ ID NO. 29: Synthetic DNA

## Claims

1. A human Nap1 protein having the amino acid sequence represented by SEQ ID NO. 2 or a protein having an amino acid sequence which includes substitution, deletion or addition of one or more amino acids of the amino acid sequence represented by SEQ ID NO. 2 and having apoptosis-suppressing activities.

2. A DNA encoding the protein according to Claim 1.

3. A DNA having the nucleotide sequence represented by SEQ ID NO. 1.

4. A DNA capable of hybridizing to the nucleotide sequence of the DNA according to Claim 2 or 3 under stringent conditions and encoding a protein having apoptosis-suppressing activities.

5. A recombinant vector containing the DNA according to any one of Claims 2 to 4 and a vector.

6. A transformant which is obtained by introducing the vector according to Claim 5 into a host cell.

7. A process for producing the said protein, which comprises culturing the transformant according to Claim 6 in a medium to produce and accumulate the protein according to Claim 1 in the culture and harvesting the protein from the culture to be obtained.

8. A therapeutic composition for Alzheimer's disease containing the protein according to Claim 1 as an active ingredient.

9. An oligonucleotide having a continuous 5 to 60-bp nucleotide sequence out of the DNA nucleotide sequences according to any one of Claims 2 to 4 or an oligonucleotide having a sequence complementary to said oligonucleotide.

10. An oligonucleotide according to Claim 9, which has a nucleotide sequence represented by SEQ ID NO. 17 or 18.

11. An oligonucleotide according to Claim 9, which has a nucleotide sequence represented by SEQ ID NO. 26 or 28.

12. A method for detecting an mRNA of a human Nap1 gene using the oligonucleotide according to Claim 9 or 10.

13. A diagnostic reagent for Alzheimer's disease containing the oligonucleotide according to Claim 9 or 10.

14. A method for repressing transcription of the human Nap1 gene or translation of mRNA thereof using the oligonucleotide according to Claim 9 or 11.

15. A therapeutic compositoin for apoptosis-participating diseases containing the oligonucleotide according to Claim 9 or 11.

16. An antibody recognizing the protein according to Claim 1.

17. An immunoassay of the protein according to Claim 1 using the antibody according to Claim 16.

18. A diagnostic reagent for Alzheimer's disease containing the antibody according to Claim 16.

19. A therapeutic composition for apoptosis-participating diseases containing the
antibody according to Claim 16 as an active ingredient.
